# EUROPÄISCHE PATENTSCHRIFT

(11) **EP 3 132 476 B1**
(45) Veröffentlichungstag und Bekanntmachung des Hinweises auf die Patenterteilung: **23.08.2023**
(21) Anmeldenummer: 15712049.4
(22) Anmeldetag: 17.03.2015
(51) Int. Cl.: H10K 85/10, H10K 85/60, H10K 50/11

(54) **MATERIALIEN FÜR ELEKTRONISCHE VORRICHTUNGEN**
MATERIALS FOR ELECTRONIC DEVICES
MATIÈRES POUR DISPOSITIFS ÉLECTRONIQUES

(30) Priorität: 16.04.2014 EP 14001391
(43) Veröffentlichungstag der Anmeldung: 22.02.2017
(73) Patentinhaber: Merck Patent GmbH, 64293 Darmstadt (DE)
(72) Erfinder: HEIL, Holger, 60389 Frankfurt am Main (DE); RODRIGUEZ, Lara-Isabel, 64283 Darmstadt (DE); BURKHART, Beate, 64293 Darmstadt (DE); MEYER, Sebastian, 63741 Aschaffenburg (DE)
(86) Internationale Anmeldenummer: PCT/EP2015/000587
(87) Internationale Veröffentlichungsnummer: WO 2015/158409

(56) Entgegenhaltungen:
- DE-A1-102007 024 850
- DE-A1-102007 024 850
- US-A1- 2004 076 853
- US-B2- 7 829 206
- JIAN CAO ET AL: "Manganese(III) Acetate-Mediated Cyclization of Diarylmethylenecyclopropa[ b ]naphthalenes: A Method for the Synthesis of 1,2-Benzanthracene Derivatives", THE JOURNAL OF ORGANIC CHEMISTRY, Bd. 76, Nr. 22, 18. November 2011 (2011-11-18), Seiten 9329-9337, XP55193236, ISSN: 0022-3263, DOI: 10.1021/jo2015906
- Jian Cao ET AL: "Manganese(III) Acetate-Mediated Cyclization of Diarylmethylenecyclopropa[ b ]naphthalenes: A Method for the Synthesis of 1,2-Benzanthracene Derivatives", The Journal of Organic Chemistry, vol. 76, no. 22, 18 November 2011 (2011-11-18), pages 9329-9337, XP055193236, ISSN: 0022-3263, DOI: 10.1021/jo2015906

## Beschreibung

Die vorliegende Anmeldung betrifft eine elektronische Vorrichtung, welche eine substituierte Benzanthracen-Verbindung enthält.

Unter dem Begriff elektronische Vorrichtung werden gemäß der vorliegenden Erfindung allgemein elektronische Vorrichtungen verstanden, welche organische Materialien enthalten. Bevorzugt werden darunter OLEDs verstanden.

Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten der elektronischen Vorrichtungen sind weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der elektronischen Vorrichtungen sowie die realisierten Farbwerte. Auch ist es für bestimmte Anwendungen wichtig, lösliche Verbindungen zur Verfügung zu haben. Insbesondere bei blau emittierenden OLEDs besteht Verbesserungspotential bezüglich der Lebensdauer der Vorrichtungen und den Farbwerten des emittierten Lichts.

Ein wichtiger Ansatzpunkt, um die genannten Verbesserungen zu erreichen, ist die Auswahl der Verbindung, die als Matrix in der emittierenden Schicht der elektronischen Vorrichtung, bevorzugt in Kombination mit einer fluoreszierenden Emitterverbindung, eingesetzt wird.

Unter einer Matrix (oder Matrixverbindung oder Matrixmaterial) in der emittierenden Schicht werden im Rahmen der vorliegenden Anmeldung solche Verbindungen verstanden, die in der emittierenden Schicht der elektronischen Vorrichtung vorhanden sind, jedoch keine Emitterverbindungen darstellen, d.h. nicht oder im Wesentlichen nicht an der Lichtemission der emittierenden Schicht beteiligt sind.

Unter Emitterverbindungen werden entsprechend Verbindungen der emittierenden Schicht verstanden, welche beim Betrieb der elektronischen Vorrichtung Licht emittieren. Vom Begriff fluoreszierende Emitter sind gemäß der vorliegenden Anmeldung Verbindungen umfasst, bei denen die Lichtemission aus einem Singulett-Zustand heraus erfolgt.

Im Stand der Technik sind als Matrixverbindungen zur Verwendung in der emittierenden Schicht eine Vielzahl von Verbindungen beschrieben. Beispiele dafür sind Anthracene mit Arylsubstituenten in 2,6,9 und 10-Position, wie beispielsweise in WO 2007/110129 oder US 2004/076853 A1 beschrieben, Bis-Anthracenverbindungen, wie beispielsweise in WO 2007/065678 beschrieben, oder Anthracenverbindungen mit voneinander verschiedenen Substituenten in 9- und in 10-Position, wie beispielsweise in EP 1 553154 beschrieben.

Weiterhin sind im Stand der Technik Benzanthracenverbindungen mit bestimmtem Substitutionsmuster für diese Verwendung beschrieben, beispielsweise in WO 2008/145239. Die dort offenbarten Benzanthracenverbindungen sind dadurch charakterisiert, dass sie in einer der Positionen 2, 3, 4, 5 oder 6 eine Aryl- oder Heteroarylgruppe tragen, und ansonsten keine weiteren Substituenten aufweisen, inbesondere keine Substituenten in den Positionen 7 und 12.

Auch wenn die in der WO 2008/145239 offenbarten Verbindungen sehr gute Eigenschaften aufweisen, besteht doch weiterhin Verbesserungsbedarf. Besonderes Interesse besteht dabei an der Entwicklung von neuen Verbindungen, die tiefblaue Farbkoordinaten des emittierten Lichts und/oder eine höhere Lebensdauer der elektronischen Vorrichtung ermöglichen. Weiterhin ist es für bestimmte Anwendungen von hohem Interesse, Verbindungen mit verbesserter Löslichkeit in gängigen organischen Lösungsmitteln zur Verfügung zu haben, insbesondere in Lösungsmitteln, die in Druckverfahren oder Spin-Coating-Verfahren zur Herstellung von elektronischen Vorrichtungen eingesetzt werden.

Auch besteht Interesse an neuartigen Verbindungen, die als Alternativen zu den im Stand der Technik bekannten Verbindungen dienen können. Wünschenswerte Verwendungen der Verbindungen beschränken sich dabei nicht auf die Verwendung als Matrix, sondern schließen auch die Verwendung als beispielsweise Elektronentransportmaterial, Lochtransportmaterial oder Emitter ein.

In Untersuchungen zu neuen Verbindungen zur Verwendung in elektronischen Vorrichtungen wurde nun unerwartet gefunden, dass Benzanthracen-Verbindungen mit einem definierten Substitutionsmuster gemäß folgender Formel (I) oder (II) hervorragend zur Verwendung in elektronischen Vorrichtungen geeignet sind. Insbesondere lösen sie eine oder mehrere, bevorzugt alle der oben genannten technischen Aufgaben Bereitstellung von OLEDs mit tiefblauen Farbkoordinaten des emittierten Lichts, Bereitstellung von OLEDs mit hoher Lebensdauer und Bereitstellung von Verbindungen mit guter Löslichkeit in organischen Lösungsmitteln.

Gegenstand der Erfindung ist in Anspruch 1 definiert.

Die grafische Darstellung 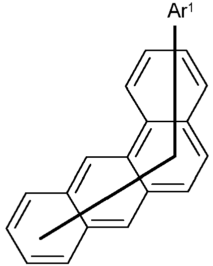 bedeutet, dass eine Gruppe Ar¹ an einer beliebigen Position ausgewählt aus den Positionen 1 bis 12 des Benzanthracens gebunden ist.

Die grafische Darstellung 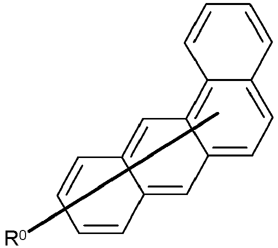 bedeutet, dass eine Gruppe R⁰ an einer Position gewählt aus den Positionen 5, 6, 7, 8, 9, 10, 11 oder 12 des Benzanthracens gebunden ist.

In der vorliegenden Anmeldung wird die folgende Nummerierung der Positionen des Benzanthracen-Gerüsts verwendet:

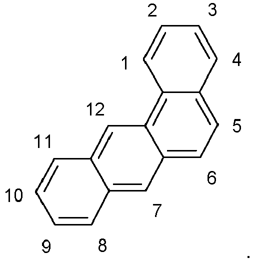

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Es ist bevorzugt, dass die Gruppe Ar¹ in einer Position des Benzanthracens gewählt aus Positionen 2, 3, 4, 5, 6, und besonders bevorzugt in einer Position gewählt aus Positionen 4 und 5.

Besonders ist Ar¹ gewählt aus der Gruppe bestehend aus Anthracen, Benzanthracen, Phenanthren, Benzphenanthren, Pyren, Chrysen, Perylen, Fluoranthen, Naphthacen, Pentacen, Benzpyren, wobei die genannten Gruppen jeweils mit einem oder mehreren Resten R² substituiert sein können.

Bevorzugt sind Reste R², die an Gruppen Ar¹ gebunden sind, gewählt aus H, D, F, CN, geradkettigen Alkyl- oder Alkoxygruppen mit 1 bis 10 C-Atomen und verzweigten oder cyclischen Alkyl- oder Alkoxygruppen mit 3 bis 10 C-Atomen, wobei die oben genannten Gruppen jeweils mit einem oder mehreren Resten R³ substituiert sein können, und aus aromatischen Ringsystemen mit 6 bis 24 aromatischen Ringatomen, die mit einem oder mehreren Resten R³ substituiert sein können, und aus heteroaromatischen Ringsystemen mit 5 bis 24 aromatischen Ringatomen, die mit einem oder mehreren Resten R³ substituiert sein können. Dabei sind die aromatischen Ringsysteme mit 6 bis 24 aromatischen Ringatomen, die mit einem oder mehreren Resten R³ substituiert sein können, und die heteroaromatischen Ringsysteme mit 5 bis 24 aromatischen Ringatomen, die mit einem oder mehreren Resten R³ substituiert sein können, bevorzugt gewählt aus der Gruppe bestehend aus Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Terphenyl, Quaterphenyl, Fluoren, Spirobifluoren, Furan, Benzofuran, Isobenzofuran, Dibenzofuran, Thiophen, Benzothiophen, Isobenzothiophen, Dibenzothiophen, Indol, Isoindol, Carbazol, Indolocarbazol, Indenocarbazol, Pyridin, Chinolin, Isochinolin, Acridin, Phenanthridin, Benzimidazol, Pyrimidin, Pyrazin, und Triazin, wobei die genannten Gruppen jeweils mit einem oder mehreren Resten R³ substituiert sein können.

Bevorzugte Ausführungsformen der Gruppe Ar¹ entsprechen den folgenden Formeln (Ar¹-1) und (Ar¹-5)

∗-Ar² Formel (Ar¹-1)

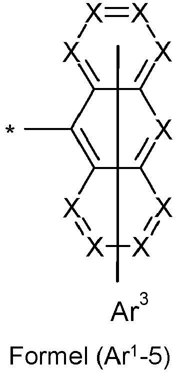

wobei gilt:
- X: ist bei jedem Auftreten gleich oder verschieden gewählt aus der Gruppe enthaltend N, CR² und C, wobei X nur dann gleich C sein kann, wenn eine Gruppe Ar³ an X gebunden ist;
- Ar³: ist gewählt aus aromatischen Ringsystemen mit 6 bis 24 aromatischen Ringatomen, die mit einem oder mehreren Resten R³ substituiert sein können, und aus heteroaromatischen Ringsystemen mit 5 bis 24 aromatischen Ringatomen, die mit einem oder mehreren Resten R³ substituiert sein können;
und die mit * markierte Bindung ist die Anbindungsposition der Gruppe an die Benzanthraceneinheit.

Der Formel (Ar¹-5) entsprechend, ist die Gruppe Ar³ jeweils in einer beliebigen freien Position des Anthracenylderivats gebunden. Bevorzugt ist für Formel (Ar¹-5) die Bindung in der 9- bzw. 10-Position des Anthracenylderivats.

Die Gruppe X ist bevorzugt gewählt aus der Gruppe enthaltend CR² und C, wobei X nur dann gleich C sein kann, wenn eine Gruppe Ar³ an X gebunden ist.

Ar² ist gewählt aus der Gruppe bestehend aus Anthracen, Benzanthracen, Phenanthren, Benzphenanthren, Pyren, Chrysen, Fluoranthen und Naphthacen, wobei die genannten Gruppen jeweils mit einem oder mehreren Resten R² substituiert sein können.

Ar³ ist bevorzugt gewählt aus Arylgruppen mit 6 bis 22 aromatischen Ringatomen, die mit einem oder mehreren Resten R³ substituiert sein können, und aus Heteroarylgruppen mit 5 bis 22 aromatischen Ringatomen, die mit einem oder mehreren Resten R³ substituiert sein können. Besonders bevorzugt ist Ar³ gewählt aus der Gruppe bestehend aus Benzol, Naphthalin, Anthracen, Benzanthracen, Phenanthren, Benzphenanthren, Pyren, Chrysen, Perylen, Fluoranthen, Naphthacen, Pentacen, Benzpyren, Benzofuran, Isobenzofuran, Dibenzofuran, Benzothiophen, Isobenzothiophen, Dibenzothiophen, Indol, Isoindol, Carbazol, Chinolin, Isochinolin, Acridin, Phenanthridin, Benzimidazol, wobei die genannten Gruppen jeweils mit einem oder mehreren Resten R³ substituiert sein können. Ganz besonders bevorzugt ist Ar³ gewählt aus der Gruppe bestehend aus Benzol, Naphthalin, Anthracen, Benzanthracen, Phenanthren, Benzphenanthren, Pyren, Chrysen, Perylen, Fluoranthen und Naphthacen, wobei die genannten Gruppen jeweils mit einem oder mehreren Resten R³ substituiert sein können.

Bevorzugte Ausführungsformen der Gruppe Ar¹ nach Formel (Ar¹-1) entsprechen den folgenden Formeln (Ar¹-1-1) bis (Ar¹-1-6) 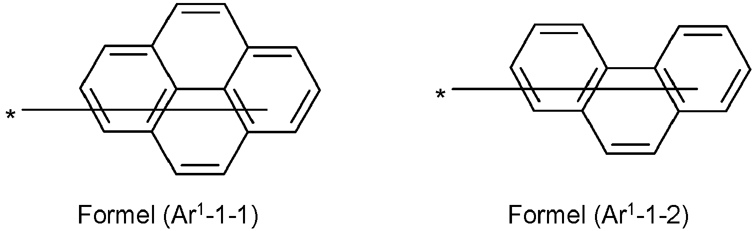 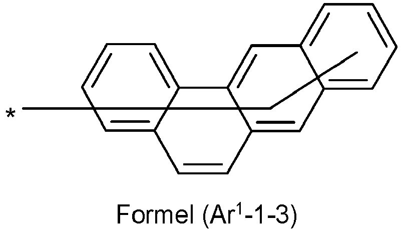 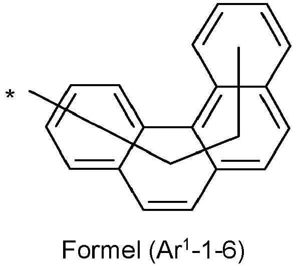 wobei gilt:
an freien Positionen können jeweils Reste R² vorhanden sein; und
die mit * markierte Bindung ist die Anbindungsposition der Gruppe an die Benzanthraceneinheit.

Bevorzugte Ausführungsformen der Gruppe Ar¹ nach Formel (Ar¹-5) entsprechen den folgenden Formeln (Ar¹-5-1) bis (Ar¹-5-7) 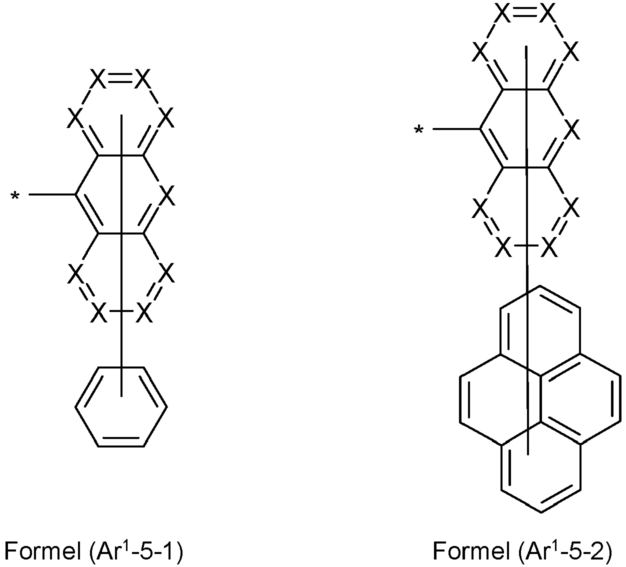 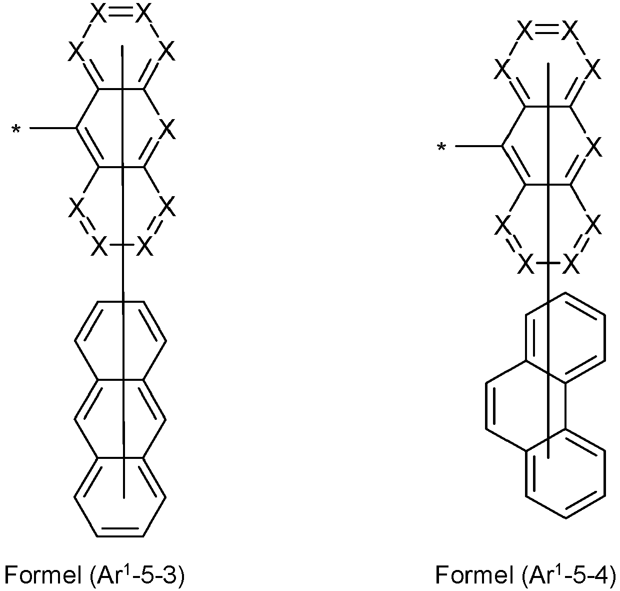 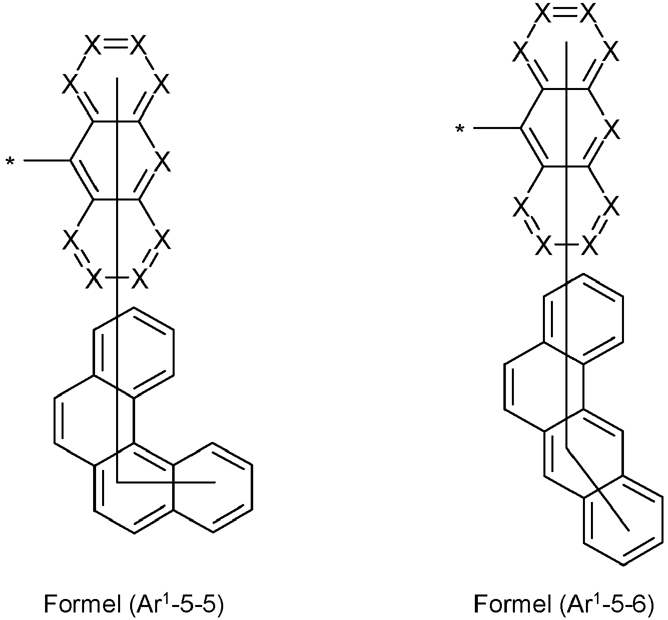 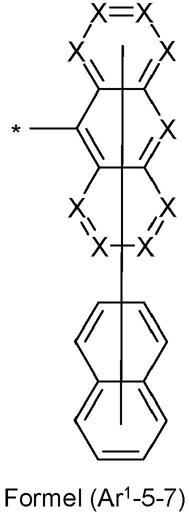 wobei gilt:
- X: ist definiert wie oben, und ist bevorzugt bei jedem Auftreten gleich oder verschieden gewählt aus CR² und C, wobei X nur C sein kann, wenn an X ein Substituent gebunden ist;

an freien Positionen können jeweils Reste R³ vorhanden sein; und
die mit * markierte Bindung ist die Anbindungsposition der Gruppe an die Benzanthraceneinheit.

Bevorzugt ist R⁰ gewählt aus Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Methoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy und 2-Ethylhexyloxy, Trimethylsilyl, Triethylsilyl, sowie mit Resten-Si(R⁴)₃ substituierten Derivaten der oben genannten Reste.

Es gilt, der Kennzeichnung in Formel (1-1-1) entsprechend, dass die Gruppe Ar¹ in Formel (I-1-1) in einer Position gewählt aus Positionen 1, 2, 3, 4, 5, 6 gebunden ist. Es ist für Formel (1-1-1) bevorzugt, dass die Gruppe Ar¹ in einer Position gewählt aus Positionen 2, 4 und 5 gebunden ist, besonders bevorzugt in einer Position gewählt aus Positionen 4 und 5.

Folgende Verbindungen sind Beispiele für Verbindungen gemäß Formel (I-1-1):
(*: Nicht erfindungsgemäße Verbindungen)

| | |
|---|---|
| 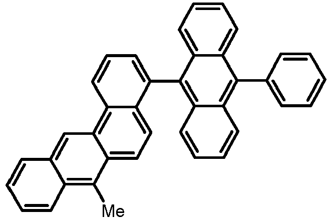 | 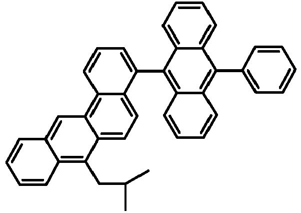 |
| 1 | 2 |
| 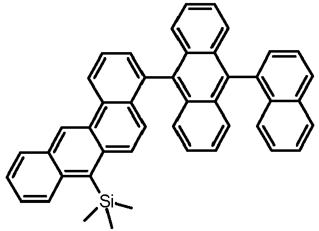 | 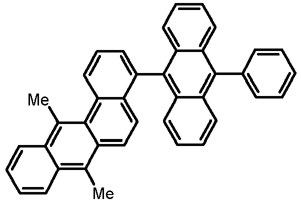 |
| 3 | 4* |
| 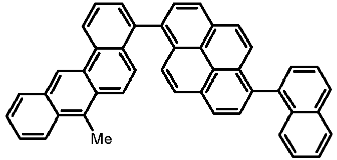 | 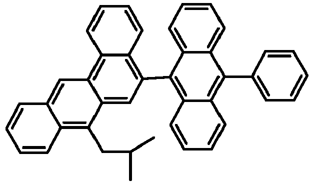 |
| 5 | 6 |
| 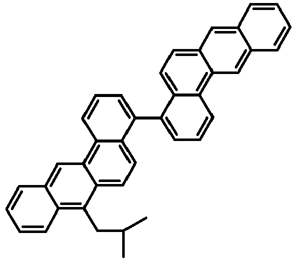 | 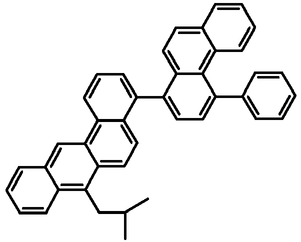 |
| 7 | 8 |
| 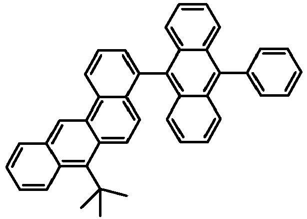 | 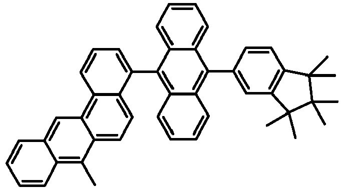 |
| 9 | 10 |
| 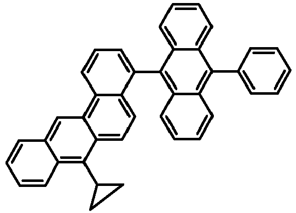 | 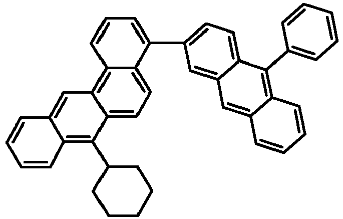 |
| 11 | 12 |
| 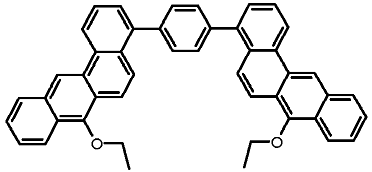 | 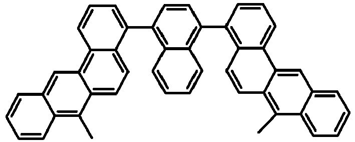 |
| 13* | 14* |
| 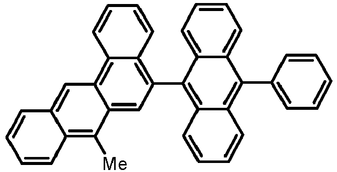 | 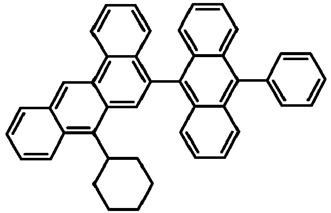 |
| 15 | 16 |
| 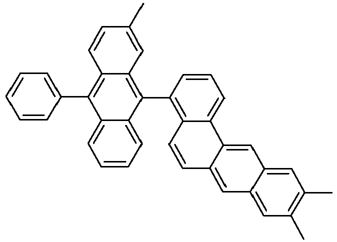 | 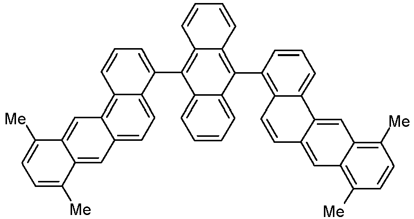 |
| 17* | 18* |
| 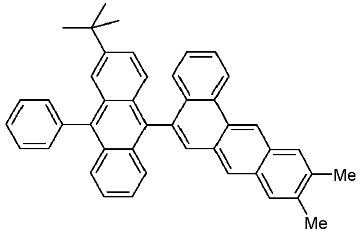 | 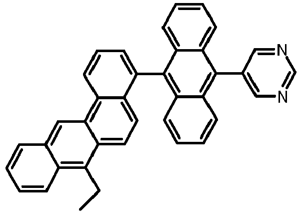 |
| 19* | 20 |
| 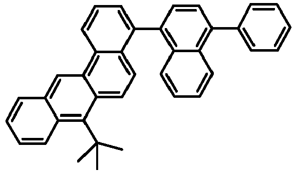 | 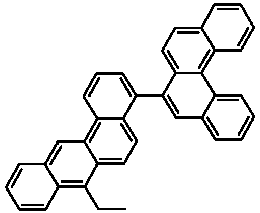 |
| 21* | 22 |
| 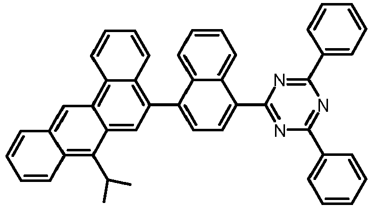 | 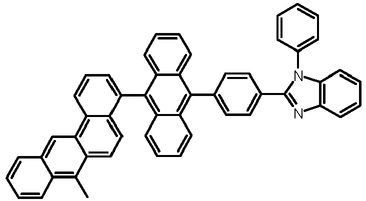 |
| 23* | 24* |
| 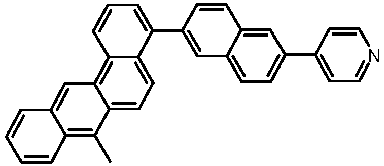 | 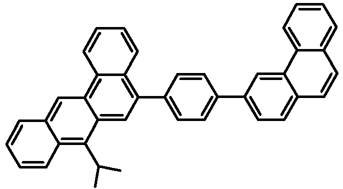 |
| 25* | 26* |
| 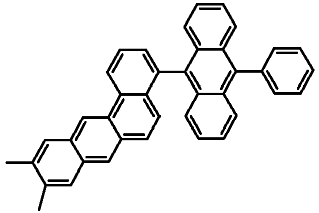 | 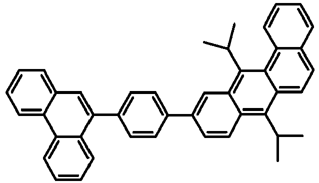 |
| 27* | 28* |
| 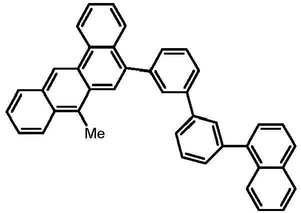 | 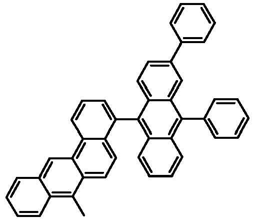 |
| 29* | 30 |
| 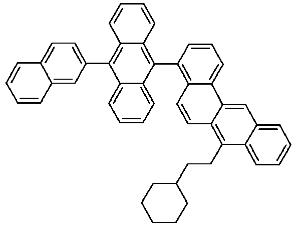 | 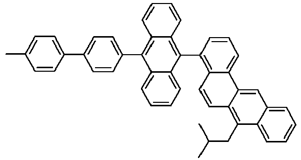 |
| 31 | 32 |
| 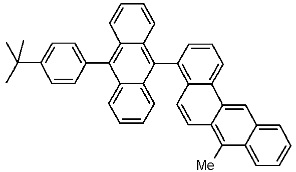 | 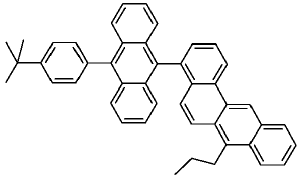 |
| 33 | 34 |
| 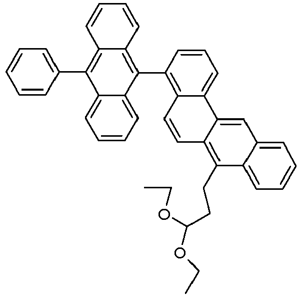 | 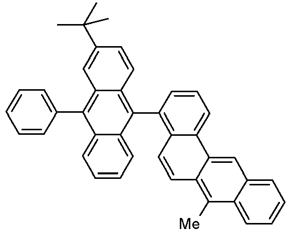 |
| 35 | 36 |
| 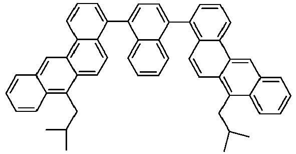 | 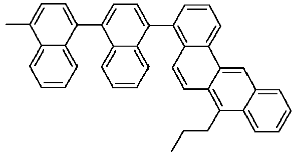 |
| 37* | 38* |
| 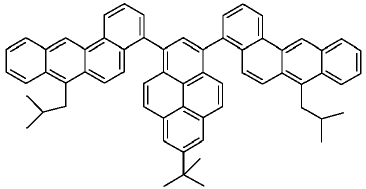 | 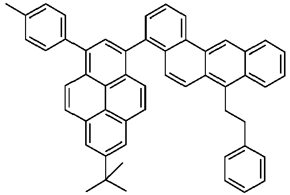 |
| 39 | 40 |
| 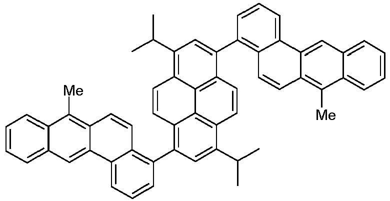 | 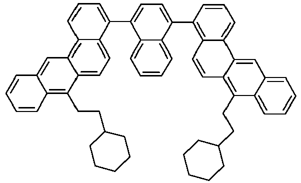 |
| 41 | 42* |
| 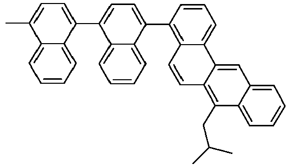 | 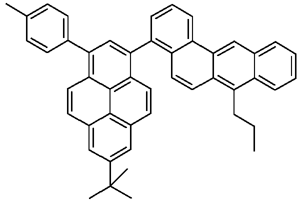 |
| 43* | 44 |
| 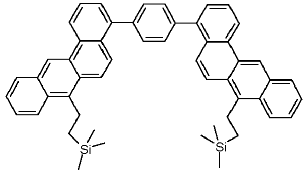 | 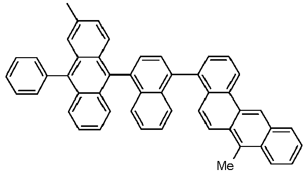 |
| 45* | 46* |
| 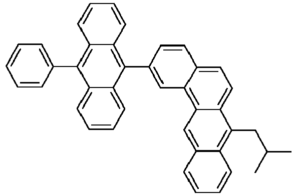 | 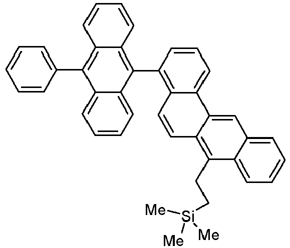 |
| 47 | 48 |
| 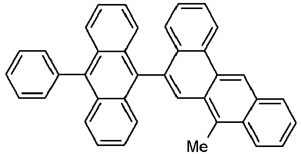 | 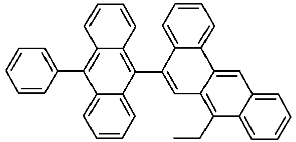 |
| 49 | 50 |
| 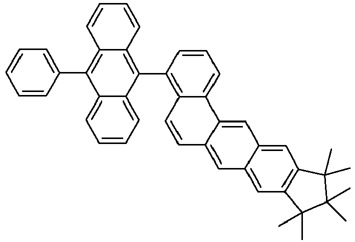 | 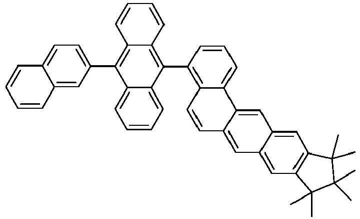 |
| 51* | 52* |
| 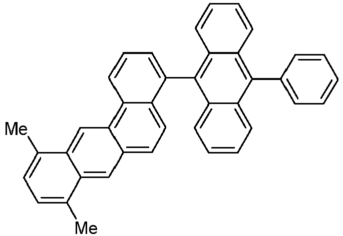 | 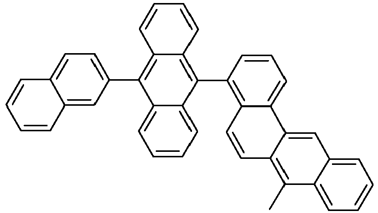 |
| 53* | 54 |

Die erfindungsgemäßen Verbindungen können beispielsweise gemäß folgendem Reaktionsschema hergestellt werden (Schema 1). 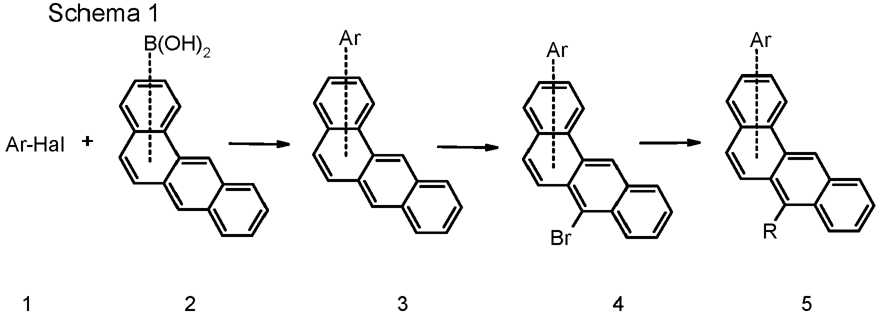
Ar: beliebiges aromatisches oder heteroaromatisches Ringsystem
Hal: Halogenatom oder andere reaktive Gruppe
R: beliebiger organischer Rest

Dazu wird von einem Boronsäure-substituierten Benzanthracenderivat 2 ausgegangen. Wie solche Verbindungen der allgemeinen Struktur 2 hergestellt werden können, ist in den Ausführungsbeispielen der WO 2008/145239 beschrieben. Die Boronsäuregruppe kann dabei in einer beliebigen Position, ausgewählt aus den Positionen 1-6 des Benzanthracens, vorliegen. Anschließend wird eine Kupplungsreaktion mit einem beliebigen aromatischen oder heteroaromatischen Ringsystem, das mit einer reaktiven Gruppe substituiert ist, durchgeführt. Dabei wird eine Verbindung der allgemeinen Struktur 3 erhalten. Diese wird anschließend in 7-Position bromiert, und es wird in 7-Position ein Rest R eingeführt. Der Rest R ist bevorzugt eine wahlweise substituierte Alkylgruppe.

Ein alternatives Syntheseschema, um zu den erfindungsgemäßen Verbindungen zu gelangen, geht von in 8-, 9- oder 11-Position mit Arylgruppen substituierten Benzanthracenderivaten aus (Schema 2). Wie solche Verbindungen hergestellt werden können, ist beispielsweise in WO 2011/012212 beschrieben. Anschließend wird, wie für Schema 2 beschrieben, in 7-Position bromiert und eine Gruppe R, bevorzugt eine wahlweise substituierte Alkylgruppe, eingeführt. 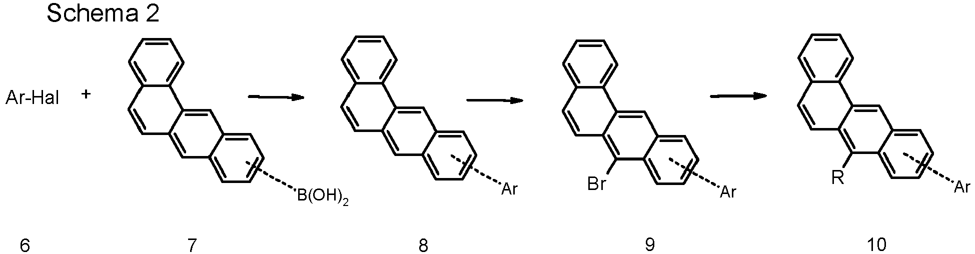
Ar: beliebiges aromatisches oder heteroaromatisches Ringsystem
Hal: Halogenatom oder andere reaktive Gruppe
R: beliebiger organischer Rest

Ein nochmals alternatives Verfahren, um zu erfindungsgemäßen Verbindungen zu gelangen, ist im folgenden Schema 3 gezeigt. 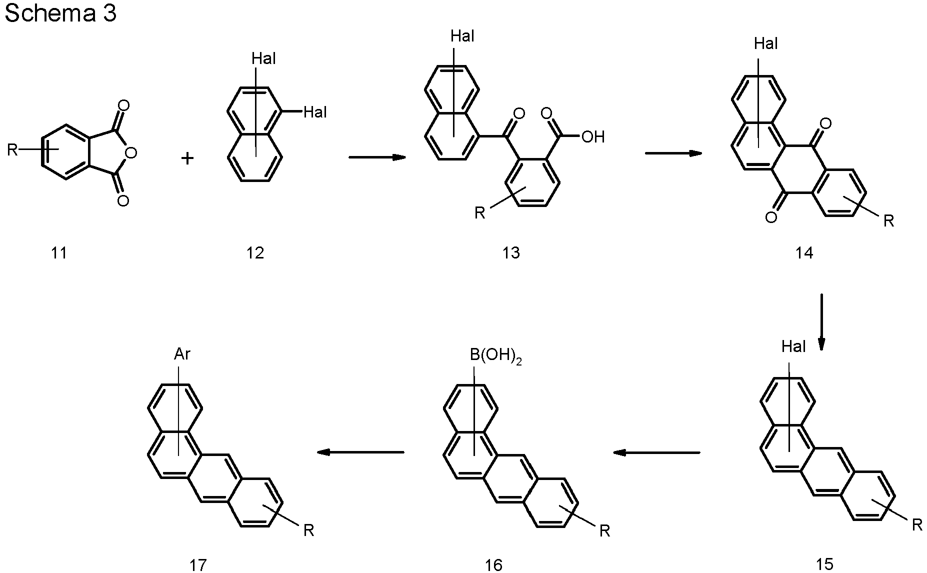
Ar: beliebiges aromatisches oder heteroaromatisches Ringsystem
Hal: Halogenatom oder andere reaktive Gruppe
R: beliebiger organischer Rest

Dazu wird zunächst ein Phthalsäureanhydrid-Derivat 11 mit einer substituierten Naphthylverbindung 12 umgesetzt. Die entstehende Verbindung 13 wird in einer intramolekularen Friedel-Crafts-Acylierung zur Chinon-Verbindung 14 weiter umgesetzt. Diese wird zu einer Benzanthracenverbindung 15 reduziert. Anschließend wird in der Position der reaktiven Gruppe Hal eine Boronsäurefunktion eingeführt, so dass eine Verbindung der Formel 16 erhalten wird. Über eine Suzuki-Reaktion wird anschließend eine Gruppe Ar eingeführt, so dass die erfindungsgemäße Verbindung 17 erhalten wird, die eine Gruppe R in einer der Positionen 8-11 des Benzanthracens aufweist.

Gegenstand der Anmeldung ist daher ein Verfahren zur Herstellung einer Verbindung der Formel (I) oder (II), umfassend die Schritte 1) bis 3) in der genannten Reihenfolge:
1) Herstellung einer Benzanthracen-Verbindung, welche mit einer oder mehreren aromatischen oder heteroaromatischen Ringsystemen substituiert ist, durch Kupplungsreaktion zwischen einem BenzanthracenDerivat und einem aromatischen oder heteroaromatischen Ringsystem;
2) Halogenierung, bevorzugt Bromierung, des Benzanthracens;
3) Einführung eines Substituenten in der halogenierten, bevorzugt bromierten, Position.

Bei diesem Verfahren findet die Halogenierung, bevorzugt Bromierung, im Schritt 2) bevorzugt in Position 7 des Benzanthracens statt. Weiterhin ist es bevorzugt, dass das durch Kupplungsreaktion in Schritt 1) eingeführte aromatische oder heteroaromatische Ringsystem in einer Position gewählt aus Positionen 1 bis 6 am Benzanthracen vorliegt.

Weiterer Gegenstand der Anmeldung ist ein Verfahren zur Herstellung einer Verbindung der Formel (I) oder (II), umfassend die folgenden Schritte in der angegebenen Reihenfolge:
I) Herstellung eines substituierten Benzanthracenderivats aus einem Naphthylderivat und einem Phthalsäureanhydrid;
II) Kupplungsreaktion des substituierten Benzanthracenderivats mit einem aromatischen oder heteroaromatischen Ringsystem.

Bevorzugt umfasst Schritt I) eine Acylierungsreaktion, eine intramolekulare Friedel-Crafts-Acylierung und eine Reduktion eines Chinonderivats. Bevorzugt liegt der Substituent des Benzanthracenderivats in einer Position des Benzanthracens gewählt aus Positionen 8-11. Bevorzugt erfolgt die Kupplungsreaktion in Schritt II) an einer Position des Benzanthracens gewählt aus Positionen 1-6, besonders bevorzugt an einer Position gewählt aus Positionen 4 und 5.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I) oder (II), wobei die Bindung(en) zum Polymer, Oligomer oder Dendrimer an beliebigen, in Formel (I) oder (II) mit R¹, R² oder R³ substituierten Positionen lokalisiert sein können. Je nach Verknüpfung der Verbindung gemäß Formel (I) oder (II) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) oder (II) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) oder (II) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) oder (II) in Oligomeren, Dendrimeren und Polymeren gelten dieselben Bevorzugungen wie oben für Verbindungen gemäß Formel (I) oder (II) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) oder (II) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder (II) oder mindestens ein Polymer, Oligomer oder Dendrimer enthaltend mindestens eine Einheit gemäß Formel (I) oder (II) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Die erfindungsgemäßen Verbindungen können in jeder Funktion in der organischen Elektrolumineszenzvorrichtung eingesetzt werden, beispielsweise, als Matrixmaterial, als emittierendes Material, als lochtransportierendes Material oder als elektronentransportierendes Material. Bevorzugt ist die Verwendung als Matrixmaterial in einer emittierenden Schicht, bevorzugt einer fluoreszierenden emittierenden Schicht, und die Verwendung als emittierendes Material, bevorzugt als fluoreszierendes emittierendes Material, in einer emittierenden Schicht einer organischen Elektrolumineszenzvorrichtung.

Weiterer Gegenstand der Erfindung ist daher die Verwendung einer Verbindung gemäß Formel (I) oder (II) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist eine elektronische Vorrichtung, enthaltend mindestens eine Verbindung der Formel (I) oder (II). Bevorzugt ist die elektronische Vorrichtung gewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht mindestens eine Verbindung gemäß Formel (I) oder (II) enthält. Ganz besonders bevorzugt ist eine organische Elektrolumineszenzvorrichtung, enthaltend Anode, Kathode und mindestens eine emittierende Schicht, enthaltend mindestens eine Verbindung gemäß Formel (I) oder (II).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Dabei müssen nicht alle der genannten Schichten vorhanden sein, und es können zusätzlich weitere Schichten vorhanden sein, beispielsweise eine Elektronenblockierschicht anodenseitig an die emittierende Schicht angrenzend, oder eine Lochblockierschicht kathodenseitig an die emittierende Schicht angrenzend.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) oder (II) enthält und wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert. Alternativ und/oder zusätzlich können in einer derartigen organischen Elektrolumineszenzvorrichtung die erfindungsgemäßen Verbindungen auch in der Lochtransportschicht oder in einer anderen Schicht vorhanden sein.

Die erfindungsgemäße Verbindung eignet sich insbesondere zur Verwendung als Matrixverbindung für eine Emitterverbindung, bevorzugt eine blau emittierende Emitterverbindung oder als Emitterverbindung, bevorzugt als blau emittierende Emitterverbindung.

Bevorzugt ist die Verwendung als Matrixverbindung für fluoreszierende Emitterverbindungen.

Die erfindungsgemäße Verbindung kann jedoch auch als Matrixverbindung für Emitterverbindungen, die thermisch aktivierte verzögerte Fluoreszenz (TADF) zeigen, verwendet werden. Grundzüge des Emissionsmechanismus bei TADF sind in H. Uoyama et al., Nature 2012, 492, 234 offenbart.

Wird die erfindungsgemäße Verbindung als Matrixmaterial eingesetzt, kann sie mit beliebigen, dem Fachmann bekannten emittierenden Verbindungen kombiniert eingesetzt werden. Bevorzugt wird sie in Kombination mit den unten angegebenen bevorzugten emittierenden Verbindungen eingesetzt, besonders den unten angegebenen bevorzugten fluoreszierenden Verbindungen.

Für den Fall, dass die emittierende Schicht der organischen Elektrolumineszenzvorrichtung eine Mischung aus einer emittierenden Verbindung und einer Matrixverbindung enthält, gilt folgendes:
Der Anteil der emittierenden Verbindung in der Mischung der emittierenden Schicht beträgt bevorzugt zwischen 0.1 und 50.0 %, besonders bevorzugt zwischen 0.5 und 20.0 %, und ganz besonders bevorzugt zwischen 1.0 und 10.0 %. Entsprechend beträgt der Anteil des Matrixmaterials bzw. der Matrixmaterialien bevorzugt zwischen 50.0 und 99.9 %, besonders bevorzugt zwischen 80.0 und 99.5 %, und ganz besonders bevorzugt zwischen 90.0 und 99.0 %.

Dabei wird unter den Angaben der Anteile in % im Rahmen der vorliegenden Anmeldung Vol.-% verstanden, wenn die Verbindungen aus der Gasphase aufgebracht werden, und es wird darunter Gew.-% verstanden, wenn die Verbindungen aus Lösung aufgebracht werden.

Wenn die erfindungsgemäße Verbindung als emittierende Verbindung in einer emittierenden Schicht eingesetzt wird, wird sie bevorzugt in Kombination mit einem oder mehreren Matrixmaterialien eingesetzt. Die bevorzugten Anteile von emittierender Verbindung und Matrixmaterial sind dabei wie oben angegeben.

Die erfindungsgemäße Verbindung kann weiterhin auch als elektronentransportierende Verbindung in einer Elektronentransportschicht, einer Lochblockierschicht oder einer Elektroneninjektionsschicht eingesetzt werden. Hier für ist es bevorzugt, dass die erfindungsgemäße Verbindung einen oder mehrere Substituenten gewählt aus elektronenarmen Heteroarylgruppen wie beispielsweise Triazin, Pyrimidin oder Benzimidazol enthält.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende emittierende Verbindungen eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden emittierenden Verbindungen können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Indenofluorene.

Bevorzugte fluoreszierende emittierende Verbindungen sind in der folgenden Tabelle abgebildet:

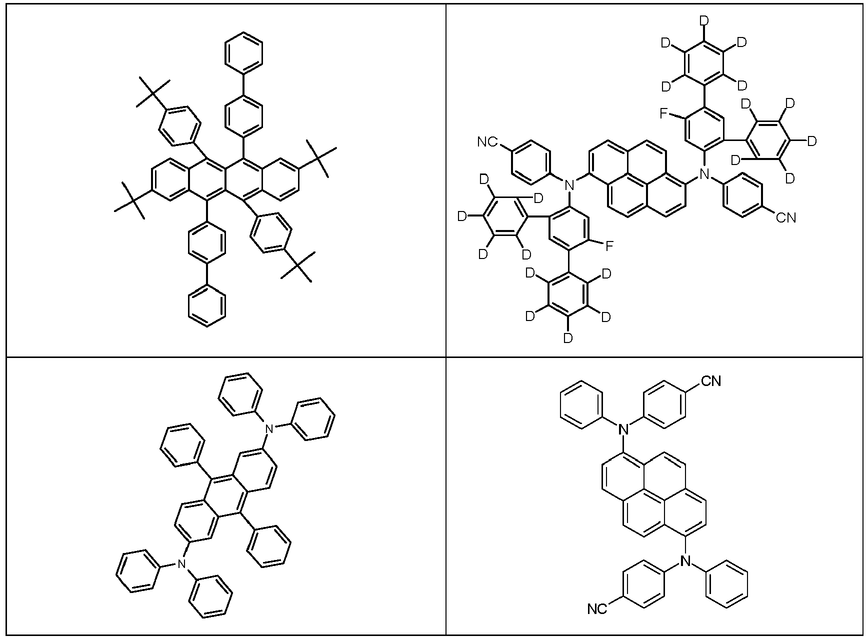

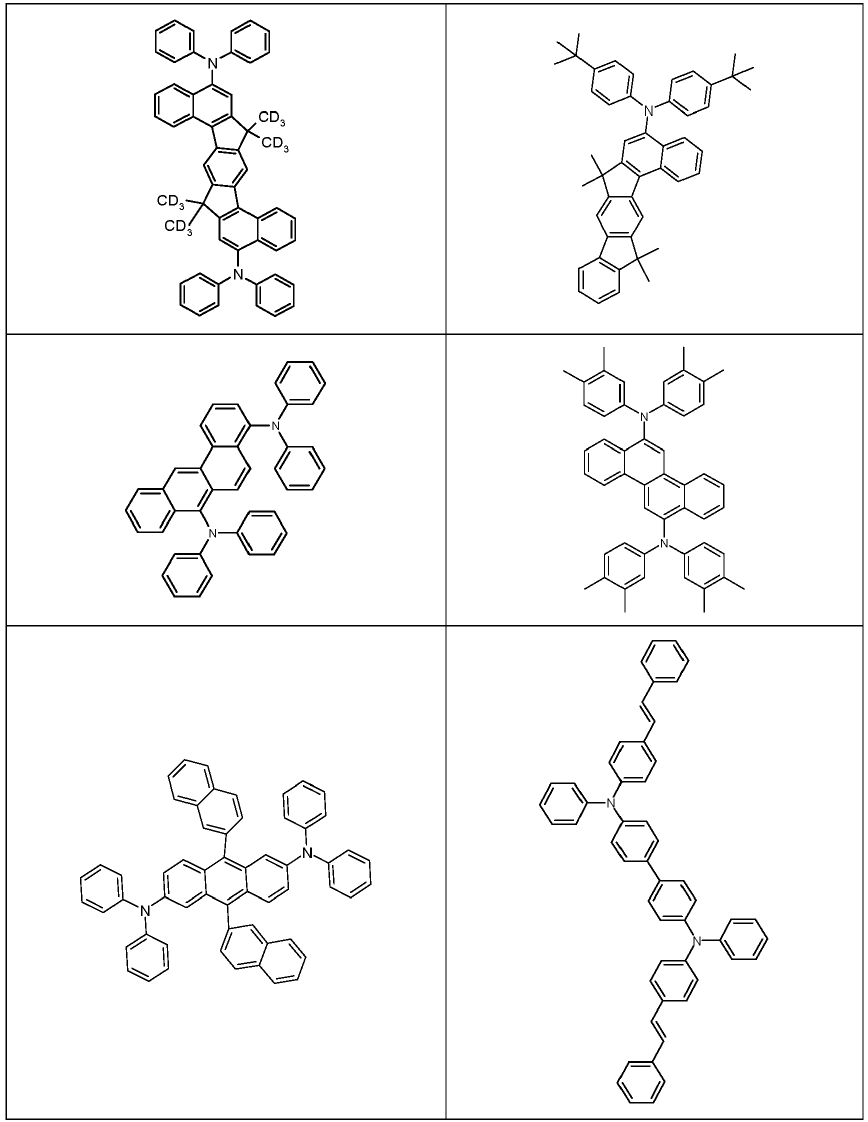

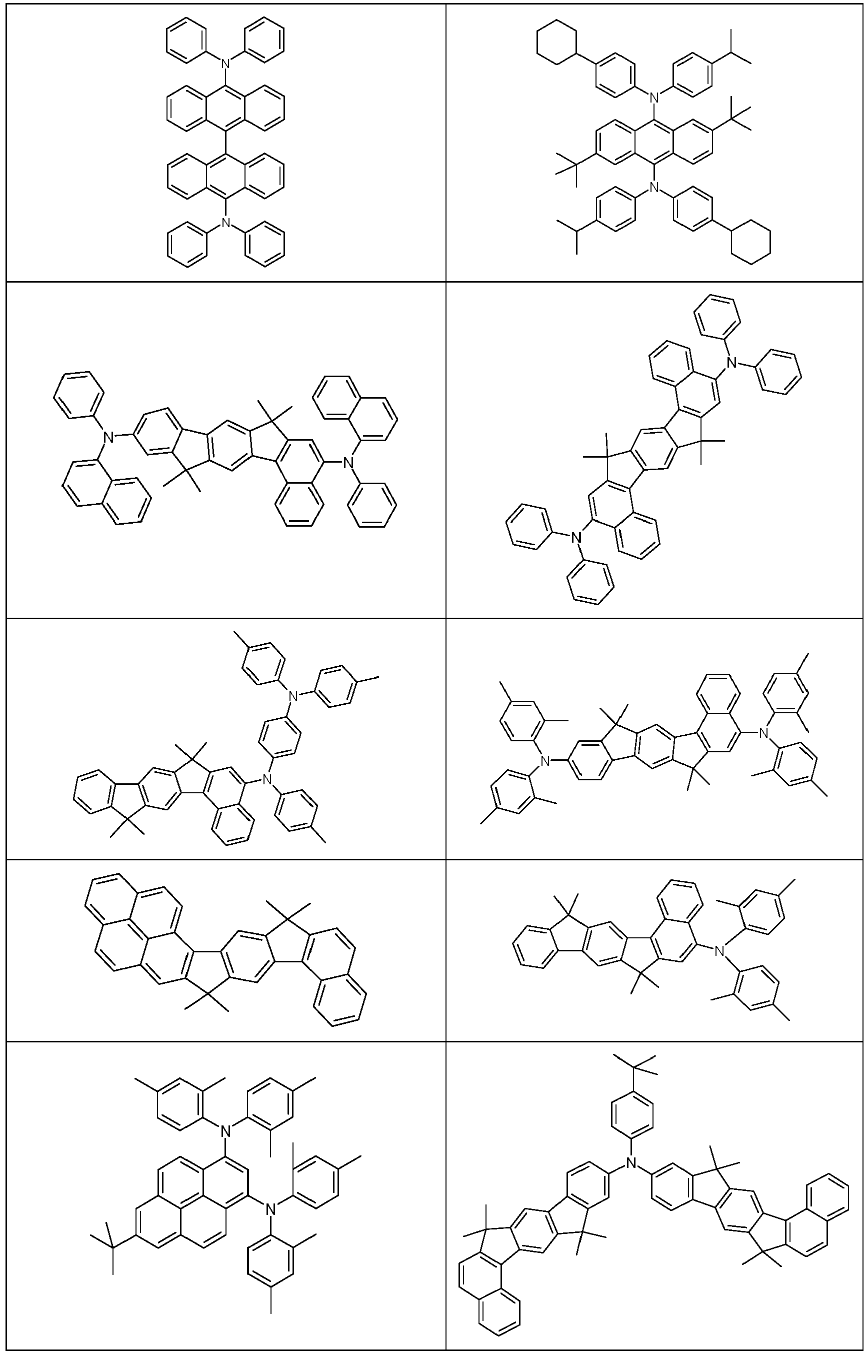

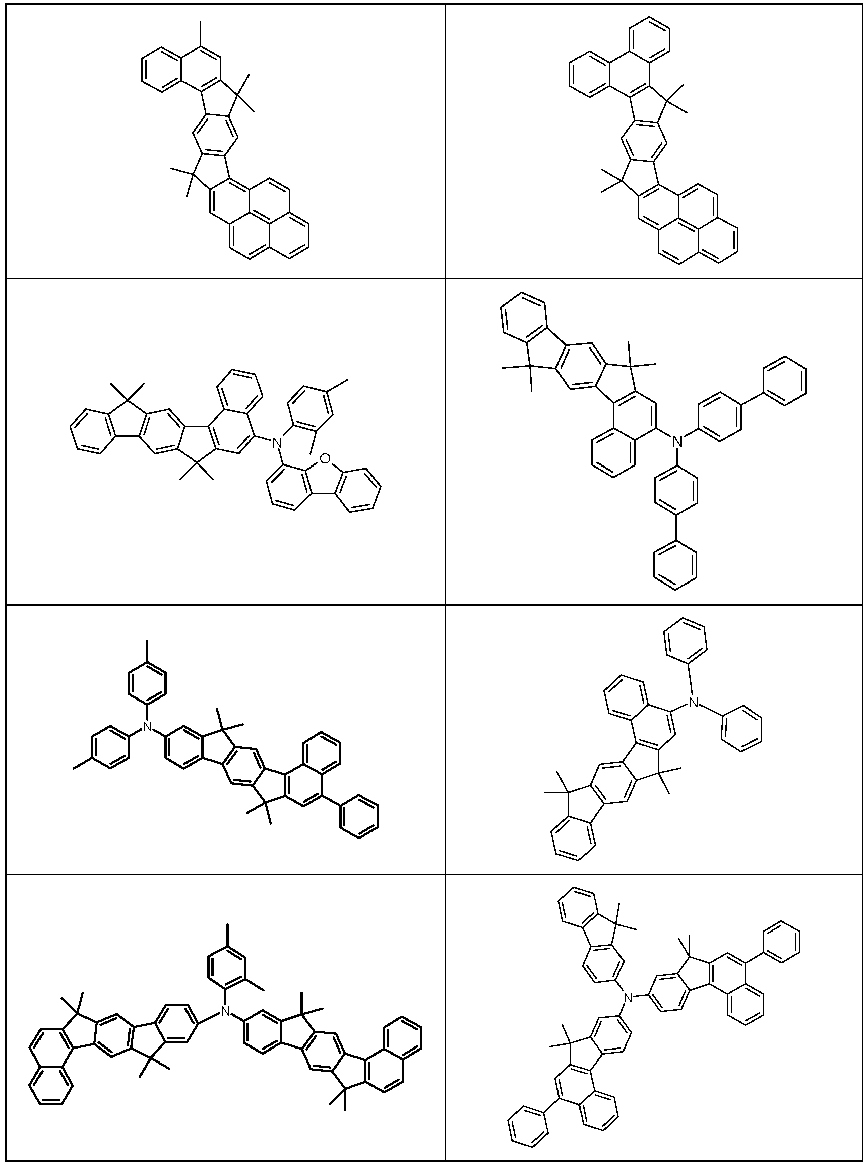

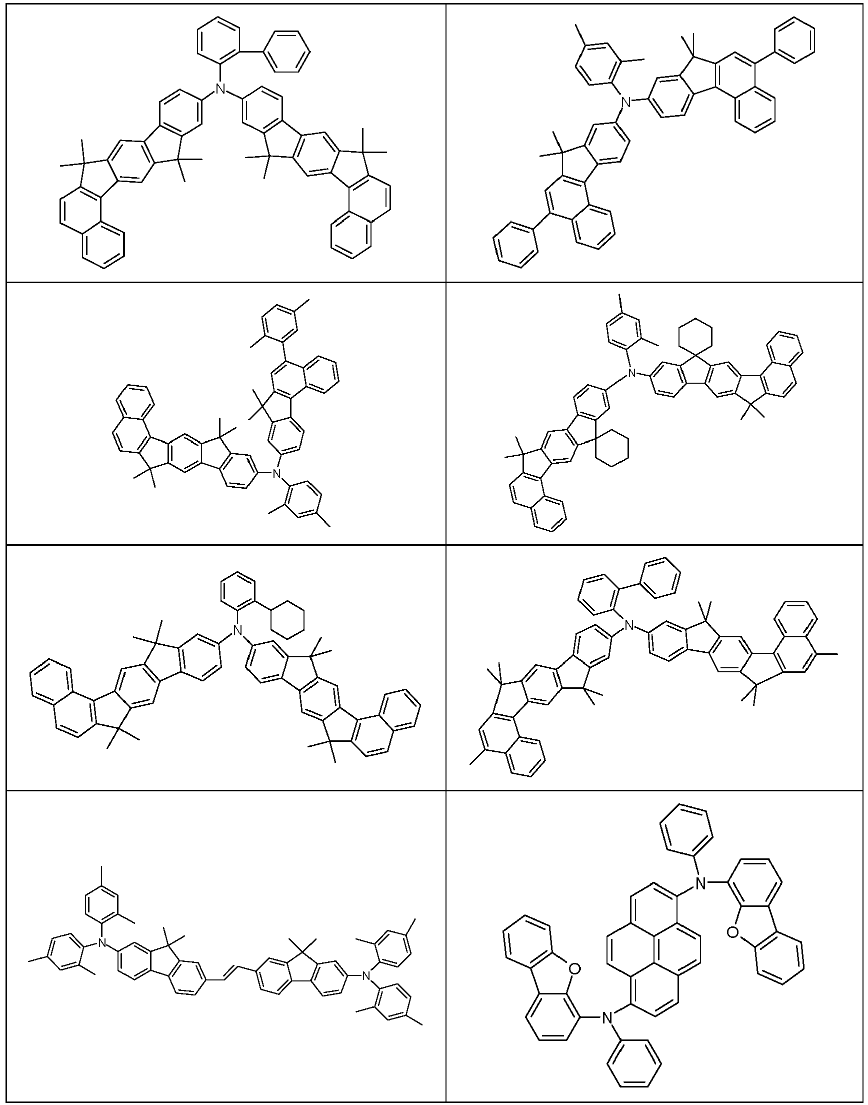

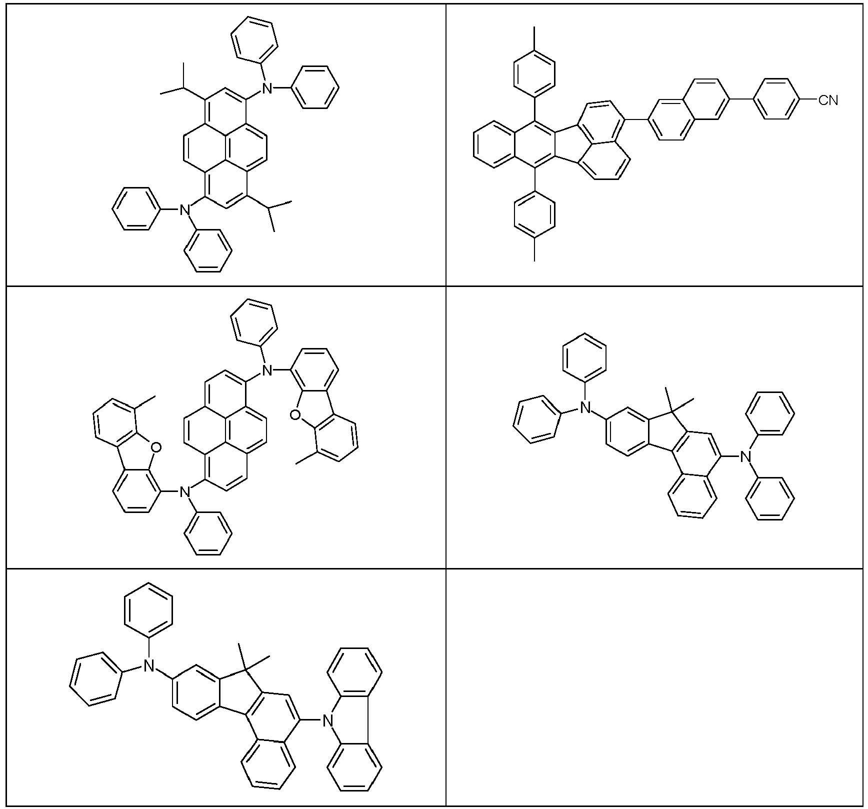

Bevorzugte Matrixmaterialien für phosphoreszierende emittierende Verbindungen sind aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729 und Diazaphosphol-Derivate, z. B. gemäß WO 2010/054730.

Bevorzugte Matrixmaterialien zur Verwendung in Kombination mit fluoreszierenden emittierenden Verbindungen sind neben den Verbindungen der Formel (I) oder (II) ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind neben der Verbindungen der Formel (I) oder (II) Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. gemäß WO 2012/150001).

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Aufgrund der guten Löslichkeit der Verbindungen gemäß Formel (I) oder (II) ist es bevorzugt, dass die Schicht enthaltend eine oder mehrere Verbindungen der Formel (I) oder (II) aus Lösung aufgebracht wird. Bevorzugt ist dies die emittierende Schicht einer organischen Elektrolumineszenzvorrichtung.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

Die erfindungsgemäßen Verbindungen werden gemäß folgendem Syntheseschema hergestellt:

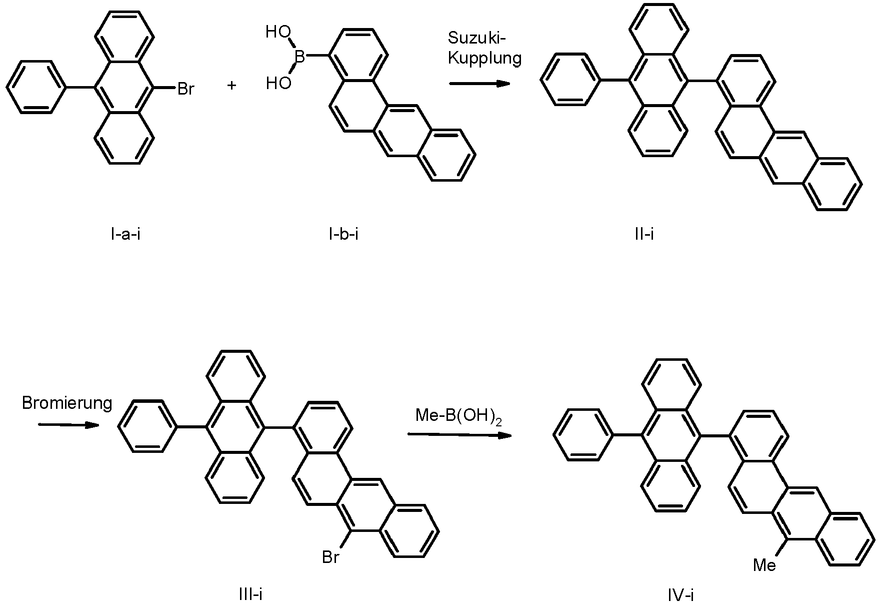

Dazu wird zunächst über eine Suzuki-Kupplung zwischen einem Benzanthracen-Boronsäure-Derivat I-b und einem Arylbromid I-a eine Benzanthracen-Arylverbindung II hergestellt (Schritt 1). Anschließend wird die Verbindung bromiert zu einer Brom-Benzanthracenverbindung III (Schritt 2). In einem letzten Schritt 3 wird über eine Suzuki-Kupplung an der Position des Brom ein Substituent eingeführt, so dass die erfindungsgemäße Verbindung IV erhalten wird.

### Schritt 1:

4-(10-Phenyl-anthracen-9-yl)-benzo[a]anthracen II-i wird gemäß folgender Literaturvorschrift synthetisiert: WO 2008/145239, Ausführungsbeispiel 8.

Analog dazu werden folgende Verbindungen hergestellt:

| Edukt I-a | Edukt I-b | Produkt II | Ausbeute |
|---|---|---|---|
| 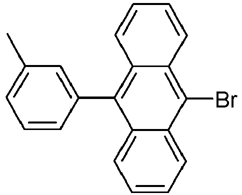 | 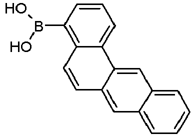 | 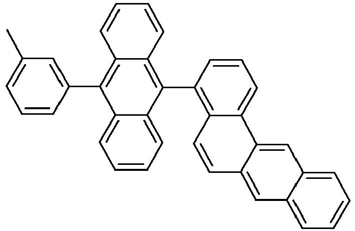 | 67% |
| 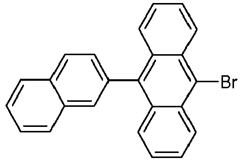 | 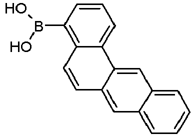 | 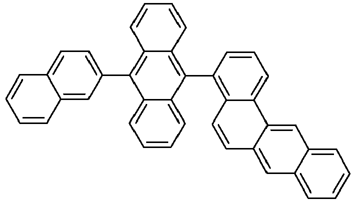 | 59% |
| 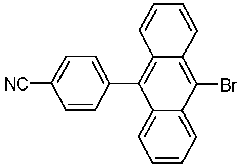 | 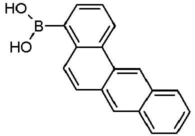 | 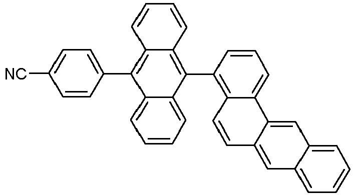 | 52% |
| 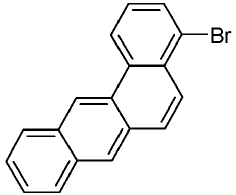 | 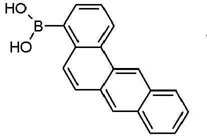 | 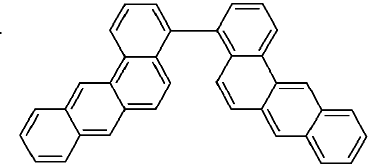 | 72% |

### Schritt 2:

### 7-Brom-4-(10-phenyl-anthracen-9-yl)-benzo[a]anthracen III-i

4-(10-Phenyl-anthracen-9-yl)-benzo[a]anthracen II-i (50 g, 104.0 mmol), *N-*bromsuccinimid (24.02 g, 135 mmol) und Benzoylperoxid (mit 25% Wasser) (12.7 ml, 20.8 mmol) werden mit 1 L Tetrahydrofuran versetzt. Der Ansatz wird über Nacht unter Rückfluss erhitzt, auf Raumtemperatur abgekühlt und mit 800 mL Chloroform und 500 mL einer 10%igen Natriumthiosulfat-Lösung erweitert. Nach Phasentrennung wird die wässrige Phase mehrmals mit Chloroform extrahiert. Die vereinigten organischen Phasen werden mit dest. Wasser gewaschen, über Magnesiumsulfat getrocknet und über Aluminiumoxid filtriert. Die organische Phase wird eingeengt. Der Rückstand wird mit Chlorobenzol zur Präzipitation gebracht und aus Heptan umkristallisiert. Man erhält Verbindung III-i als hellgelben Feststoff: 58.2 g (87% d. Th.)

Als Alternative können NBS/HBr oder Brom (katalytisch) als Bromquelle benutzt werden. Um Überbromierung zu vermeiden, wurden Reaktionen bei niedriger Temperatur durchgeführt (z.B. -10 °C).

Analog dazu werden folgende Verbindungen hergestellt:

| Edukt II | Produkt III | Ausbeute |
|---|---|---|
| 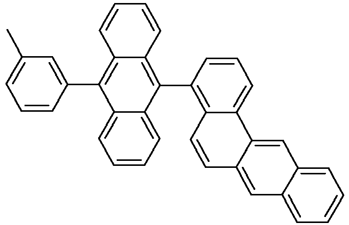 | 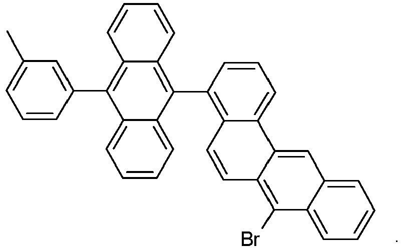 | 53% |
| 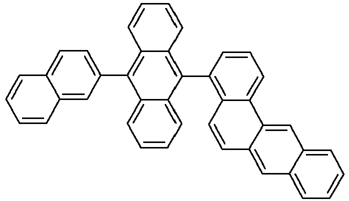 | 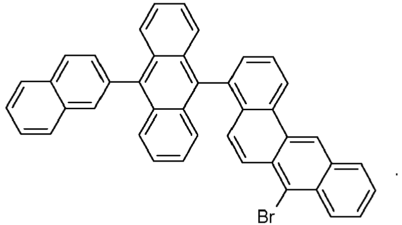 | 64% |
| 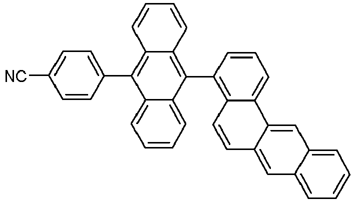 | 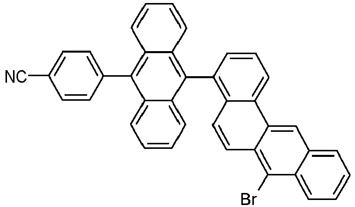 | 68% |
| 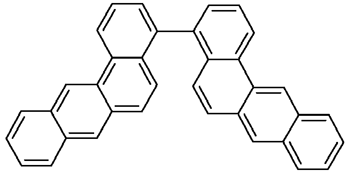 | 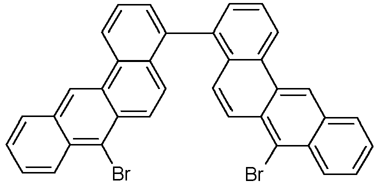 | 64% |

### Schritt 3:

### 7-Methyl-4-(10-phenyl-anthracen-9-yl)-benzo[a]anthracen IV-i

5.47 g (97%, 88.6 mmol) Methylboronsäure, 25 g (44.3 mmol) 7-Brom-4-(10-phenyl-anthracen-9-yl)-benzo[a]anthracen III-i und 20.4 g (88.6 mmol) K₃RO₄*H₂O werden in 500 mL Toluol suspendiert. Zu dieser Suspension werden 1.09 g (2.66 mmol) Dicyclohexyl-(2',6'-dimethoxy-biphenyl-2-yl)-phosphan (S-Phos) und 0.3 g (1.33 mmol) Palladiumacetat gegeben, und die Reaktionsmischung wird 16 h unter Rückfluss erhitzt. Nach Erkalten wird die Reaktionsmischung mit Wasser verdünnt, die organische Phase abgetrennt, dreimal mit 100 mL Wasser gewaschen und anschließend zur Trockene eingeengt. Nach Filtration des Rohproduktes über Kieselgel mit Toluol wird der verbleibende Rückstand aus Toluol/Heptan umkristallisiert. Die Ausbeute beträgt 17.3 g (79% d. Th.).

Analog dazu werden folgende erfindungsgemäße Verbindungen IV hergestellt:

| Nr. | Edukt III | Boronsäure oder BoronsäureEster | Produkt IV | Ausbeute |
|---|---|---|---|---|
| 1 | 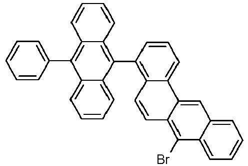 | 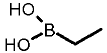 | 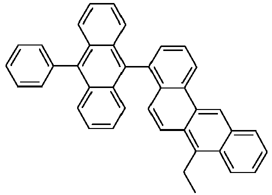 | 64% |
| 2 | 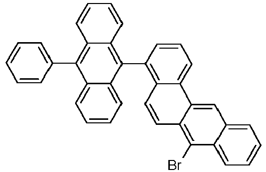 | 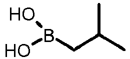 | 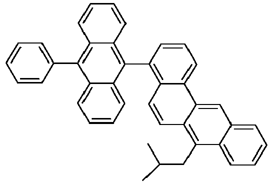 | 76% |
| 3 | 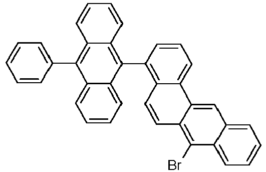 | 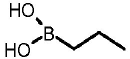 | 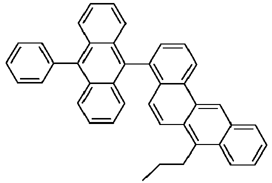 | 69% |
| 4 | 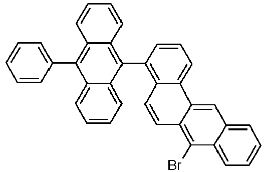 | 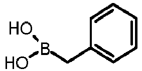 | 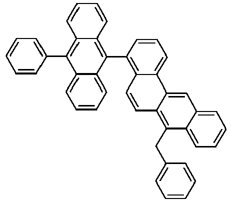 | 58% |
| 5 | 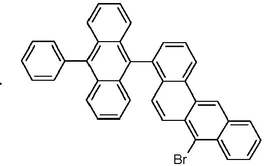 | 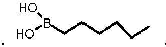 | 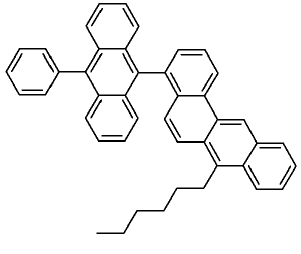 | 47% |
| 6 | 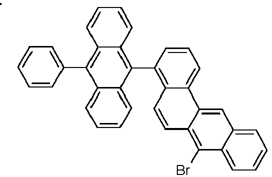 | 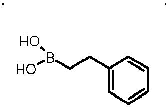 | 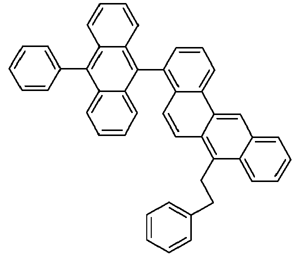 | 65% |
| 7 | 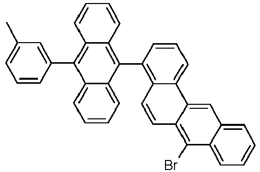 | Me-B(OH)₂ | 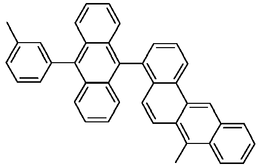 | 59% |
| 8 | 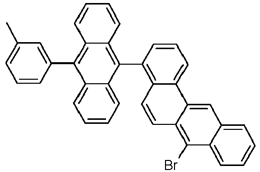 | 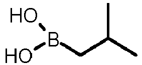 | 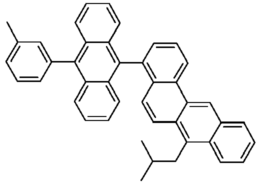 | 62% |
| 9 | 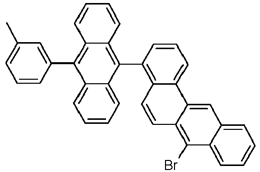 | 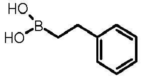 | 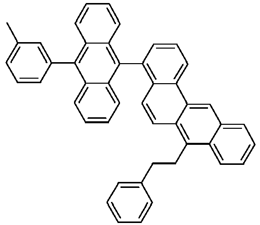 | 72% |
| 10 | 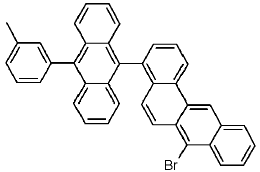 | 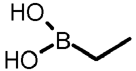 | 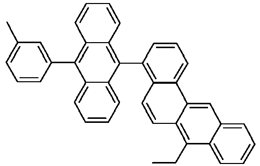 | 74% |
| 11 | 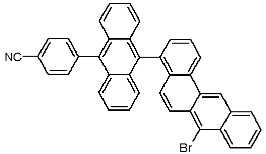 | 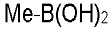 | 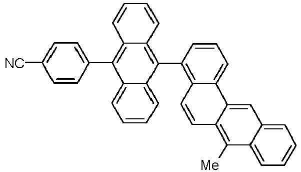 | 52% |
| 12 | 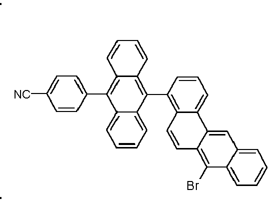 | 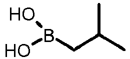 | 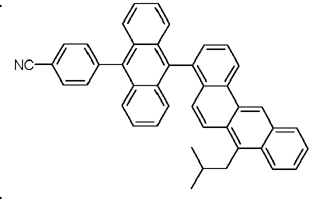 | 38% |
| 13 | 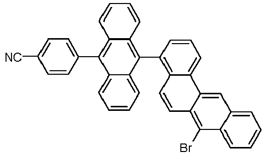 | 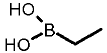 | 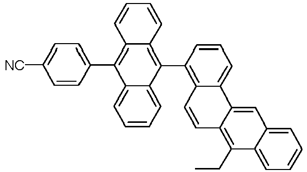 | 47% |
| 14 | 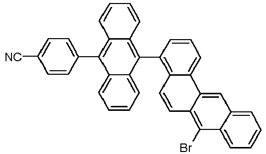 | 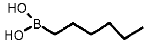 | 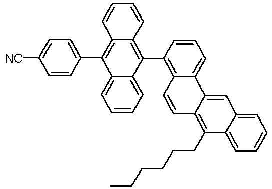 | 43% |
| 15 | 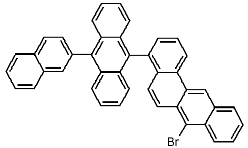 | 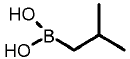 | 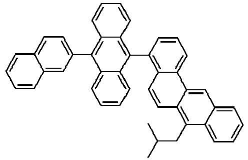 | 76% |
| 16 | 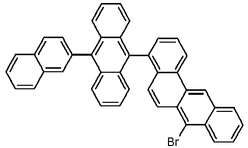 | 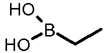 | 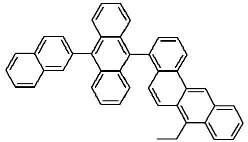 | 68% |
| 17 | 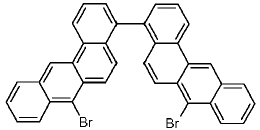 | 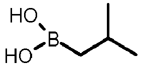 | 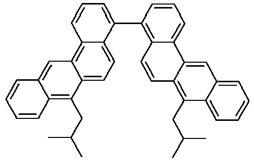 | 73% |

### B) Device-Beispiele

### B-1) Devicebeispiele aus der Gasphase: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

Als Substrate werden Glassubstrate verwendet, die mit strukturiertem ITO (Indium Zinn Oxid) der Dicke 50 nm beschichtet sind. Als Bufferschicht wird eine 20nm dicke Schicht Clevios P VP AI 4083 (bezogen von Heraeus Clevios GmbH, Leverkusen) durch Spincoating aufgebracht. Alle restlichen Materialien werden in einer Vakuumkammer thermisch aufgedampft.

Der verwendete Aufbau A ist wie folgt:
- Substrat,
- ITO (50 nm),
- Bufferschicht (20 nm),
- Lochinjektionsschicht (HTL1 95%, HIL 5%) (20 nm),
- Lochtransportschicht (HTL1) (20 nm),
- Emissionsschicht (95% Host, 5% Dotand) (20 nm),
- Elektronentransportschicht (50% ETL+50% EIL) (30 nm),
- Elektroneninjektionsschicht (EIL) (3 nm),
- Kathode (Al) (100 nm).

Die verwendeten Materialien sind in Tabelle 1 gezeigt.

Die Emissionsschicht (EML) besteht immer aus mindestens einem Matrixmaterial (Host=H) und einem emittierenden Dotierstoff (Dotand=D), der dem Matrixmaterial durch Coverdampfung in einem bestimmten Volumenanteil beigemischt wird. Eine Angabe wie H-1:D1 (95%:5%) bedeutet hierbei, dass das Material H-1 in einem Volumenanteil von 95% und D1 in einem Anteil von 5% in der Schicht vorliegt.

Die OLEDs werden standardmäßig charakterisiert. Hierfür werden die Elektrolumineszenzspektren aufgenommen, die Stromeffizienz (gemessen in cd/A) und die externe Quanteneffizienz (EQE, gemessen in Prozent) in Abhängigkeit der Leuchtdichte unter Annahme einer lambertschen Abstrahlcharakteristik aus Strom-Spannungs-Leuchtdichte-Kennlinien (IUL-Kennlinien) berechnet und abschließend die Lebensdauer der Bauteile bestimmt. Die Elektrolumineszenzspektren werden bei einer Leuchtdichte von 1000 cd/m² aufgenommen und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². Die Lebensdauer LD95 @ 1000 cd/m² ist die Zeit, die vergeht, bis die Starthelligkeit von 1000 cd/m² um 5% gesunken ist. Die erhaltenen Daten für die verschiedenen OLEDs sind in Tabelle 2 zusammengefasst.

Insbesondere eignen sich die erfindungsgemäßen Verbindungen als Matrixmaterialien in blau fluoreszierenden OLEDs (s. Beispiele V1-V5 und E6-E11). Als Vergleich dienen zwei Standard-Matrix-Materialien VH-1 und VH-2 jeweils mit einem der dunkelblau fluoreszierenden Dotanden D1, D2 und D3. Als erfindungsgemäße Verbindungen werden die Matrizes H-1 und H-2 gezeigt. Diese werden ebenfalls in Kombination mit einem der Dotanden D1, D2 und D3 verwendet.

| **Tabelle 1: Strukturen der verwendeten Materialien** | |
|---|---|
| 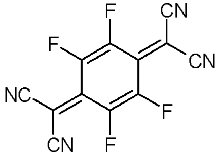 | 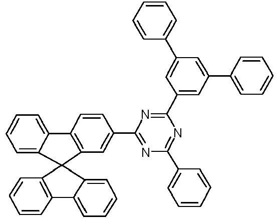 |
| HIL | ETL |
| 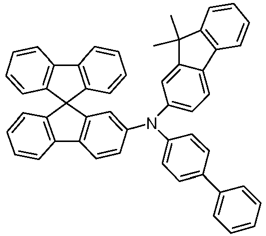 | 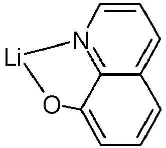 |
| HTL1 | EIL |
| 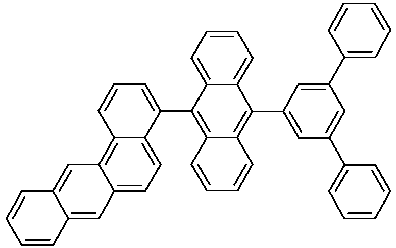 | 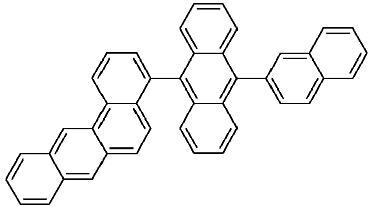 |
| VH-1 | VH-2 |
| | 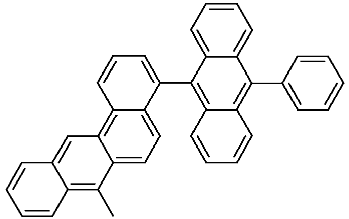 |
| | H-1 |
| 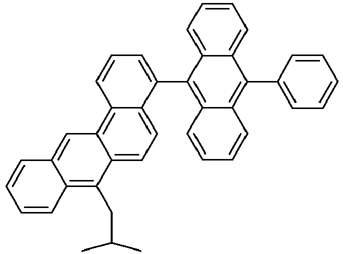 | 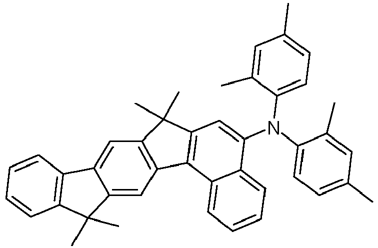 |
| H-2 | D1 |
| 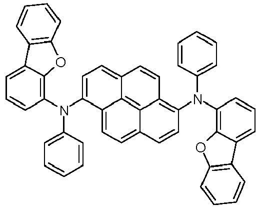 | 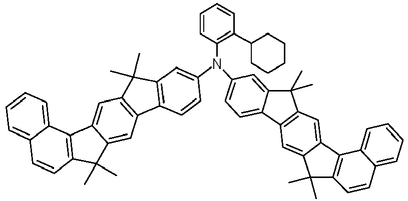 |
| D2 | D3 |

| **Tabelle 2: Daten der OLEDs** | | | | | | |
|---|---|---|---|---|---|---|
| ***Beispiel*** | ***Host*** | ***Dotand*** | ***EQE* @ *1000 cd*/*m²*** | ***LD95* @ *1000cd*/*m²*** | ***CIE*** | |
| | *95%* | *5%* | % | *[h]* | *x* | *y* |
| *V1* | *VH-1* | *D1* | *8.0* | *90* | 0.136 | 0.145 |
| *V2* | *VH-1* | *D2* | *7.5* | *100* | 0.134 | 0.101 |
| *V3* | *VH-1* | *D3* | *6.6* | 10 | 0.142 | 0.086 |
| *V4* | *VH-2* | *D1* | *7.9* | *100* | 0.135 | 0.160 |
| *V5* | *VH-2* | *D3* | *6.9* | *30* | 0.144 | 0.082 |
| *E6* | *H-1* | *D1* | *8.3* | *150* | *0.134* | 0.147 |
| *E7* | *H-1* | *D2* | *8.6* | 170 | *0.145* | 0.099 |
| *E8* | *H-1* | *D3* | *7.0* | *110* | *0.144* | 0.084 |
| *E9* | *H-2* | *D1* | *8.2* | *140* | 0.137 | 0.141 |
| *E10* | *H-2* | *D2* | *8.5* | *150* | *0.145* | 0.093 |
| *E11* | *H-2* | *D3* | *6.8* | *90* | *0.148* | 0.076 |

Die Beispiele E6 bis E11 zeigen in einer Vergleichsbetrachtung mit den Vergleichsbeispielen V1 bis V5, dass die erfindungsgemäßen Verbindungen H-1 und H-2 im Vergleich zu den Vergleichsmaterialien VH-1 und VH-2 eine verbesserte externe Quanteneffizienz (EQE) sowie eine erhöhte Lebensdauer (LD95) bei vergleichbar tiefblauer Emission erzielen.

### B-2) Devicebeispiele aus Lösung prozessiert: Herstellung der OLEDs

Die Herstellung lösungsbasierter OLEDs ist in der Literatur grundsätzlich beschrieben, z.B. in der WO 2004/037887 und der WO 2010/097155. Bei den folgenden Beispielen wurden beide Herstellungsverfahren (Aufbringung aus Gasphase und Lösungsprozessierung) kombiniert, so dass bis einschließlich Emissionsschicht aus Lösung prozessiert wurde und die darauffolgenden Schichten (Lochblockierschicht / Elektronentransportschicht) im Vakuum aufgedampft wurden. Die vorbeschriebenen allgemeinen Verfahren werden dafür wie folgt auf die hier beschriebenen Gegebenheiten (Schichtdickenvariation, Materialien) angepasst und kombiniert.

Der verwendete Aufbau B ist damit wie folgt:
- Substrat,
- ITO (50 nm),
- PEDOT (20 nm),
- Lochtransportschicht (HIL2) (20 nm),
- Emissionsschicht (92% Host, 8% Dotand) (60 nm),
- Elektronentransportschicht (ETL 50% + EIL 50%) (20 nm),
- Kathode (Al).

Als Substrat werden Glasplättchen, die mit strukturiertem ITO (Indium-Zinn-Oxid) der Dicke 50 nm beschichtet sind, verwendet. Zur besseren Prozessierung werden diese mit dem Buffer (PEDOT) Clevios P VP AI 4083 (Heraeus Clevios GmbH, Leverkusen) beschichtet oben steht PEDOT. Das Aufschleudern erfolgt an Luft aus Wasser. Die Schicht wird anschließend für 10 Minuten bei 180°C ausgeheizt. Auf die so beschichteten Glasplättchen werden die Lochtransport- sowie die Emissionsschicht aufgebracht. Bei der Lochtransportschicht handelt es sich um das Polymer der in Tabelle 3 gezeigten Struktur, das gemäß WO2010/097155 synthetisiert wurde. Das Polymer wird in Toluol gelöst, so dass die Lösung typischerweise einen Feststoffgehalt von ca. 5 g/l besitzt, wenn, wie hier, die für ein Device typische Schichtdicke von 20 nm mittels Spincoating erzielt werden soll. Die Schichten werden in einer Inertgasatmosphäre, im vorliegenden Fall Argon, aufgeschleudert und 60 min bei 180°C ausgeheizt.

Die Emissionsschicht setzt sich immer aus mindestens einem Matrixmaterial (Hostmaterial, Wirtsmaterial) und einem emittierenden Dotierstoff (Dotand, Emitter) zusammen. Eine Angabe wie H-1 (92%) : D1 (8%) bedeutet hierbei, dass das Material H-1 in einem Gewichtsanteil von 92% und der Dotand D1 in einem Gewichtsanteil von 8% in der Emissionsschicht vorliegt. Die Mischung für die Emissionsschicht wird in Toluol gelöst. Der typische Feststoffgehalt solcher Lösungen liegt bei ca. 18 g/l, wenn, wie hier, die für ein Device typische Schichtdicke von 60 nm mittels Spincoating erzielt werden soll. Die Schichten werden in einer Inertgasatmosphäre, im vorliegenden Fall Argon, aufgeschleudert und 10 Minuten bei 140°C ausgeheizt. Die verwendeten Materialien sind in Tabelle 3 gezeigt.

Die Materialien für die Elektronentransportschicht sowie für die Kathode werden in einer Vakuumkammer thermisch aufgedampft. Dabei kann z.B. die Elektronentransportschicht aus mehr als einem Material bestehen, die einander durch Co-Verdampfung in einem bestimmten Volumenanteil beigemischt werden. Eine Angabe wie ETM:EIL (50%:50%) bedeutet hierbei, dass die Materialien ETM und EIL in einem Volumenanteil von je 50% in der Schicht vorliegen. Die im vorliegenden Fall verwendeten Materialien sind in Tabelle 1 gezeigt.

| **Tabelle 3: Strukturen der verwendeten Materialien** | |
|---|---|
| 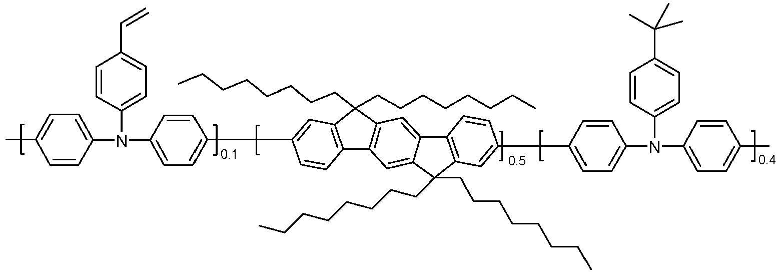 | |
| HIL2 | |
| 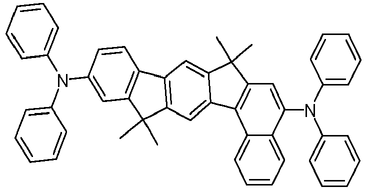 | |
| D4 | |
| 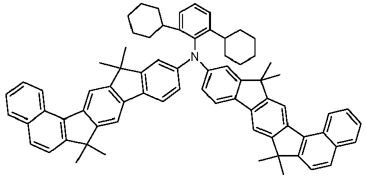 | 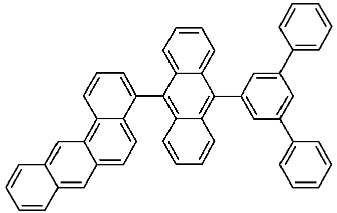 |
| D5 | VH-1 |
| 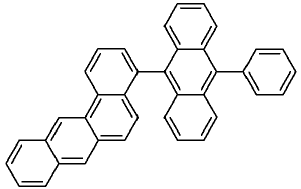 | 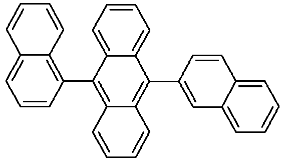 |
| VH-3 | VH-4 |
| 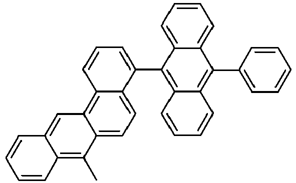 | 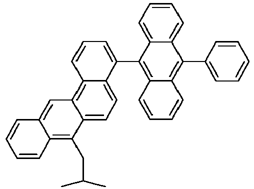 |
| H-1 | H-2 |

| **Tabelle 4: Daten der OLEDs** | | | | | | | | |
|---|---|---|---|---|---|---|---|---|
| ***Beispiel*** | ***Host*** | ***Dotand*** | ***EQE* @ *1000* cd/m²** | ***LD80* @ *10mAl cm²*** | ***CIE*** | | Löslich keit | Tg |
| | *92%* | *8%* | *%* | *[h]* | *x* | Y | gll | °C |
| *V6* | *VH-1* | *D4* | *4.8* | *200* | 0.142 | 0.211 | 70 | 175 |
| *V7* | *VH-1* | *D5* | *3.2* | *40* | 0.142 | 0.111 | 70 | 175 |
| *V8* | *VH-3* | *D4*/*D5* | *X* | *X* | X | X | <1 | 148 |
| *V9* | *VH-4* | *D4* | *4.8* | *210* | 0.136 | 0.195 | 44 | 125 |
| *V10* | *VH-4* | *D5* | *3.4* | 50 | 0.145 | 0.119 | 44 | 125 |
| *E11* | *H-1* | *D4* | *5.0* | *280* | 0.137 | 0.201 | 40 | 152 |
| *E12* | *H-1* | *D5* | *3.6* | *80* | 0.147 | 0.125 | 40 | 152 |
| *E13* | *H-2* | *D4* | *4.8* | *300* | 0.135 | 0.197 | 45 | 143 |
| *E14* | *H-2* | D5 | *3.5* | 90 | 0.146 | 0.120 | 45 | 143 |

In den Beispielen der Tabelle 4 werden die erfindungsgemäßen Verbindungen H-1 und H-2 als Hostverbindungen für die Dotanden D4 und D5 gezeigt. Als Vergleich werden die Verbindungen gemäß dem Stand der Technik VH-1, VH-3 und VH-4, ebenfalls in Kombination mit den Dotanden D4 und D5, gezeigt.

Die Ergebnisse in Tabelle 4 zeigen, dass neben einer verbesserten externe Quanteneffizienz eine deutlich verbesserte Lebensdauer (LD80) bei tiefblauer Emission sowohl im Vergleich zu VH-1 als auch zu VH-4 erzielt werden kann. Referenzmaterial VH-3 kann aufgrund der geringen Löslichkeit überhaupt nicht aus Lösung prozessiert werden (Beispiel V8; X = nicht bestimmt).

Die gefundenen Strukturen eignen sich somit neben dem Aufdampfverfahren auch für Lösungsprozessierung und führen zu hervorragenden Leistungsdaten.

## Patentansprüche

1. Verbindung einer Formel (I-1-1) 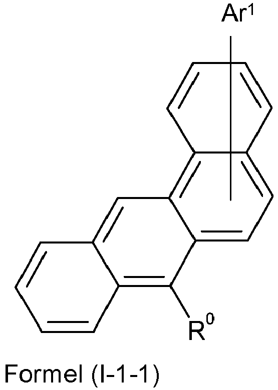 wobei für die auftretenden Symbole gilt:
Ar¹ ist eine kondensierte Arylgruppe mit 14 bis 18 artomatischen Ringatomen, die mit einem oder mehreren Resten R² substituiert sein können;
R° ist Si(R³)₃, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können;
R² ist bei jedem Auftreten gleich oder verschieden H, D, F, CN, Si(R³)₃, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können, oder ein aromatisches Ringsystem mit 6 bis 24 aromatischen Ringatomen, das mit einem oder mehreren Resten R³ substituiert sein kann, oder ein heteroaromatisches Ringsystem mit 5 bis 24 aromatischen Ringatomen, das mit einem oder mehreren Resten R³ substituiert sein kann;
R³ ist bei jedem Auftreten gleich oder verschieden H, D, F, CN, Si(R⁴)₃, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R⁴ substituiert sein können, oder ein aromatisches Ringsystem mit 6 bis 24 aromatischen Ringatomen, das mit einem oder mehreren Resten R⁴ substituiert sein kann, oder ein heteroaromatisches Ringsystem mit 5 bis 24 aromatischen Ringatomen, das mit einem oder mehreren Resten R⁴ substituiert sein kann;
R⁴ ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch D, F oder CN ersetzt sein können;

2. Verbindung nach Anspruch 1, **dadurch gekennzeichnet, dass** die Gruppe Ar¹ in einer Position gewählt aus Positionen 2, 3, 4, 5 und 6 am Benzanthracen gebunden ist.

3. Verbindung nach einem oder mehreren der Ansprüche 1 bis 3, **dadurch gekennzeichnet, dass** Ar¹ gewählt ist aus Anthracen, Benzanthracen, Phenanthren, Benzphenanthren, Pyren, Chrysen, Perylen, Fluoranthen, Naphthacen, Pentacen, Benzpyren, wobei die genannten Gruppen jeweils mit einem oder mehreren Resten R² substituiert sein können.

4. Verfahren zur Herstellung einer Verbindung gemäß einem oder mehreren der Ansprüche 1 bis 3, umfassend
A) die Schritte 1) bis 3) in der genannten Reihenfolge:
1) Herstellung einer Benzanthracen-Verbindung, welche mit einem oder mehreren aromatischen oder heteroaromatischen Ringsystemen substituiert ist, durch Kupplungsreaktion zwischen einem Benzanthracen-Derivat und einem aromatischen oder heteroaromatischen Ringsystem;
2) Halogenierung, bevorzugt Bromierung, des Benzanthracens;
3) Einführung eines Substituenten in der halogenierten, bevorzugt bromierten, Position; oder umfassend
B) die Schritte I) und II) in der angegebenen Reihenfolge:
I) Herstellung eines substituierten Benzanthracenderivats aus einem Naphthylderivat und einem Phthalsäureanhydrid;
II) Kupplungsreaktion des substituierten Benzanthracenderivats mit einem aromatischen oder heteroaromatischen Ringsystem.

5. Oligomer, Polymer oder Dendrimer enthaltend eine oder mehrere Verbindungen nach einem oder mehreren der Ansprüche 1 bis 3, wobei die Bindung(en) zum Polymer, Oligomer oder Dendrimer an beliebigen, in Formel (I) mit R¹, R² oder R³ substituierten Positionen lokalisiert sein können.

6. Formulierung, enthaltend mindestens eine Verbindung nach einem oder mehreren der Ansprüche 1 bis 3 oder mindestens ein Oligomer, Polymer oder Dendrimer nach Anspruch 5, sowie mindestens ein Lösungsmittel.

7. Elektronische Vorrichtung, ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 3 oder mindestens ein Oligomer, Polymer oder Dendrimer nach Anspruch 5.

8. Elektronische Vorrichtung nach Anspruch 7, ausgewählt aus organischen Elektrolumineszenzvorrichtungen, enthaltend Anode, Kathode, emittierende Schicht und optional weitere organische Schichten, **dadurch gekennzeichnet, dass** die mindestens eine Verbindung oder das mindestens eine Oligomer, Polymer oder Dendrimer als Matrixverbindung in Kombination mit einer oder mehreren Emitterverbindungen in der emittierenden Schicht vorliegt.

9. Verwendung einer Verbindung nach einem oder mehreren der Ansprüche 1 bis 3 oder eines Oligomers, Polymers oder Dendrimers nach Anspruch 5 in einer elektronischen Vorrichtung.

## Claims

1. Compound of a formula (1-1-1) 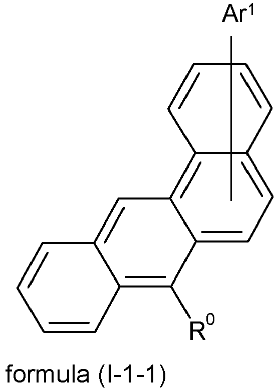 where the following applies to the symbols occurring:
Ar¹ is a condensed aryl group having 14 to 18 aromatic ring atoms, which may be substituted by one or more radicals R²;
R° is Si(R³)₃, a straight-chain alkyl or alkoxy group having 1 to 10 C atoms or a branched or cyclic alkyl or alkoxy group having 3 to 10 C atoms, where the above-mentioned groups may in each case be substituted by one or more radicals R³;
R² is on each occurrence, identically or differently, H, D, F, CN, Si(R³)₃, a straight-chain alkyl or alkoxy group having 1 to 10 C atoms or a branched or cyclic alkyl or alkoxy group having 3 to 10 C atoms, where the above-mentioned groups may in each case be substituted by one or more radicals R³, or an aromatic ring system having 6 to 24 aromatic ring atoms, which may be substituted by one or more radicals R³, or a heteroaromatic ring system having 5 to 24 aromatic ring atoms, which may be substituted by one or more radicals R³;
R³ is on each occurrence, identically or differently, H, D, F, CN, Si(R⁴)₃, a straight-chain alkyl or alkoxy group having 1 to 10 C atoms or a branched or cyclic alkyl or alkoxy group having 3 to 10 C atoms, where the above-mentioned groups may in each case be substituted by one or more radicals R⁴, or an aromatic ring system having 6 to 24 aromatic ring atoms, which may be substituted by one or more radicals R⁴, or a heteroaromatic ring system having 5 to 24 aromatic ring atoms, which may be substituted by one or more radicals R⁴;
R⁴ is on each occurrence, identically or differently, H, D, F, CN or an aliphatic, aromatic or heteroaromatic organic radical having 1 to 20 C atoms, in which, in addition, one or more H atoms may be replaced by D, F or CN.

2. Compound according to Claim 1, **characterised in that** the group Ar¹ is bonded in a position selected from positions 2, 3, 4, 5 and 6 on the benzanthracene.

3. Compound according to one or more of Claims 1 to 3, **characterised in that** Ar¹ is selected from anthracene, benzanthracene, phenanthrene, benzophenanthrene, pyrene, chrysene, perylene, fluoranthene, naphthacene, pentacene, benzopyrene, where the said groups may in each case be substituted by one or more radicals R².

4. Process for the preparation of a compound according to one or more of Claims 1 to 3, comprising
A) steps 1) to 3) in the said sequence:
1) preparation of a benzanthracene compound which is substituted by one or more aromatic or heteroaromatic ring systems by a coupling reaction between a benzanthracene derivative and an aromatic or heteroaromatic ring system;
2) halogenation, preferably bromination, of the benzanthracene;
3) introduction of a substituent in the halogenated, preferably brominated, position; or comprising
B) steps I) and II) in the sequence indicated:
I) preparation of a substituted benzanthracene derivative from a naphthyl derivative and a phthalic anhydride;
II) coupling reaction of the substituted benzanthracene derivative with an aromatic or heteroaromatic ring system.

5. Oligomer, polymer or dendrimer containing one or more compounds according to one or more of Claims 1 to 3, where the bond(s) to the polymer, oligomer or dendrimer may be localised at any desired positions in formula (1-1-1) that are substituted by R¹, R² or R³.

6. Formulation comprising at least one compound according to one or more of Claims 1 to 3 or at least one oligomer, polymer or dendrimer according to Claim 5 and at least one solvent.

7. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), containing at least one compound according to one or more of Claims 1 to 3 or at least one oligomer, polymer or dendrimer according to Claim 5.

8. Electronic device according to Claim 7, selected from organic electroluminescent devices, comprising anode, cathode, emitting layer and optionally further organic layers, **characterised in that** the at least one compound or the at least one oligomer, polymer or dendrimer is present in the emitting layer as matrix compound in combination with one or more emitter compounds.

9. Use of a compound according to one or more of Claims 1 to 3 or an oligomer, polymer or dendrimer according to Claim 5 in an electronic device.

## Revendications

1. Composé de formule (1-1-1) 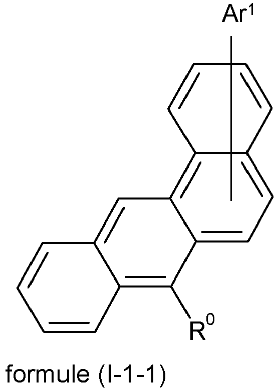 dans laquelle ce qui suit s'applique aux symboles présents :
Ar¹ est un groupement aryle condensé ayant de 14 à 18 atomes de cycle aromatique, qui peut être substitué par un ou plusieurs radicaux R² ;
R⁰ est Si(R³)₃, un groupement alkyle ou alcoxy à chaîne linéaire ayant de 1 à 10 atomes de C ou un groupement alkyle ou alcoxy ramifié ou cyclique ayant de 3 à 10 atomes de C, où les groupements susmentionnés peuvent dans chaque cas être substitués par un ou plusieurs radicaux R³ ;
R² est à chaque occurrence, de manière identique ou différente, H, D, F, CN, Si(R³)₃, un groupement alkyle ou alcoxy à chaîne linéaire ayant de 1 à 10 atomes de C ou un groupement alkyle ou alcoxy ramifié ou cyclique ayant de 3 à 10 atomes de C, où les groupements susmentionnés peuvent dans chaque cas être substitués par un ou plusieurs radicaux R³, ou un noyau aromatique ayant de 6 à 24 atomes de cycle aromatique, qui peut être substitué par un ou plusieurs radicaux R³, ou un noyau hétéroaromatique ayant de 5 à 24 atomes de cycle aromatique, qui peut être substitué par un ou plusieurs radicaux R³ ;
R³ est à chaque occurrence, de manière identique ou différente, H, D, F, CN, Si(R⁴)₃, un groupement alkyle ou alcoxy à chaîne linéaire ayant de 1 à 10 atomes de C ou un groupement alkyle ou alcoxy ramifié ou cyclique ayant de 3 à 10 atomes de C, où les groupements susmentionnés peuvent dans chaque cas être substitués par un ou plusieurs radicaux R⁴, ou un noyau aromatique ayant de 6 à 24 atomes de cycle aromatique, qui peut être substitué par un ou plusieurs radicaux R⁴, ou un noyau hétéroaromatique ayant de 5 à 24 atomes de cycle aromatique, qui peut être substitué par un ou plusieurs radicaux R⁴ ;
R⁴ est à chaque occurrence, de manière identique ou différente, H, D, F, CN ou un radical organique aliphatique, aromatique ou hétéroaromatique ayant de 1 à 20 atomes de C, où, de plus, un ou plusieurs atomes de H peuvent être remplacés par D, F ou CN.

2. Composé selon la revendication 1, **caractérisé en ce que** le groupement Ar¹ est fixé au niveau d'une position choisie parmi les positions 2, 3, 4, 5 et 6 sur le benzanthracène.

3. Composé selon l'une ou plusieurs parmi les revendications 1 à 3, **caractérisé en ce que** Ar¹ est choisi parmi anthracène, benzanthracène, phénanthrène, benzophénanthrène, pyrène, chrysène, pérylène, fluoranthène, naphtacène, pentacène, benzopyrène, où lesdits groupements peuvent dans chaque cas être substitués par un ou plusieurs radicaux R².

4. Procédé de préparation d'un composé selon l'une ou plusieurs parmi les revendications 1 à 3, comprenant
A) les étapes 1) à 3) selon ladite séquence :
1) la préparation d'un composé de benzanthracène qui est substitué par un ou plusieurs noyaux aromatiques ou hétéroaromatiques par une réaction de couplage entre un dérivé de benzanthracène et un noyau aromatique ou hétéroaromatique ;
2) l'halogénation, préférablement la bromation, du benzanthracène ;
3) l'introduction d'un substituant au niveau de la position halogénée, préférablement bromée ; ou comprenant
B) les étapes I) et II) selon la séquence indiquée :
I) la préparation d'un dérivé de benzanthracène substitué à partir d'un dérivé de naphtyle et d'un anhydride phtalique ;
II) une réaction de couplage du dérivé de benzanthracène substitué avec un noyau aromatique ou hétéroaromatique.

5. Oligomère, polymère ou dendrimère contenant un ou plusieurs composés selon l'une ou plusieurs parmi les revendications 1 à 3, dans lequel la ou les liaisons au polymère, à l'oligomère ou au dendrimère peuvent être localisées au niveau de positions quelconques dans la formule (I-1-1) qui sont substituées par R¹, R² ou R³.

6. Formulation comprenant au moins un composé selon l'une ou plusieurs parmi les revendications 1 à 3 ou au moins un oligomère, polymère ou dendrimère selon la revendication 5 et au moins un solvant.

7. Dispositif électronique choisi dans le groupe constitué par les circuits intégrés organiques (OIC), les transistors organiques à effet de champ (OFET), les transistors organiques à couche mince (OTFT), les transistors organiques émetteurs de lumière (OLET), les cellules solaires organiques (OSC), les détecteurs optiques organiques, les photorécepteurs organiques, les dispositifs organiques à extinction de champ (OFQD), les cellules électrochimiques organiques émettrices de lumière (OLEC), les diodes laser organiques (O-lasers) et les dispositifs électroluminescents organiques (OLED), contenant au moins un composé selon l'une ou plusieurs parmi les revendications 1 à 3 ou au moins un oligomère, polymère ou dendrimère selon la revendication 5.

8. Dispositif électronique selon la revendication 7, choisi parmi les dispositifs électroluminescents organiques, comprenant une anode, une cathode, une couche émettrice et éventuellement d'autres couches organiques, **caractérisé en ce que** le au moins un composé ou le au moins un oligomère, polymère ou dendrimère est présent dans la couche émettrice comme composé de matrice en combinaison avec un ou plusieurs composés émetteurs.

9. Utilisation d'un composé selon l'une ou plusieurs parmi les revendications 1 à 3 ou d'un oligomère, polymère ou dendrimère selon la revendication 5 dans un dispositif électronique.
